(19) Europäisches Patentamt
European Patent Office
Office européen des brevets

(11) Numéro de publication : **0 344 068 B1**

(12) **FASCICULE DE BREVET EUROPEEN**

(45) Date de publication du fascicule du brevet : **10.03.93 Bulletin 93/10**

(51) Int. Cl.$^5$ : **C07K 9/00,** C07K 17/10, C08B 37/00, A61K 37/00

(21) Numéro de dépôt : **89401421.6**

(22) Date de dépôt : **24.05.89**

# (54) N-Polyosyl-polypeptides.

(30) Priorité : **24.05.88 FR 8806892**

(43) Date de publication de la demande : **29.11.89 Bulletin 89/48**

(45) Mention de la délivrance du brevet : **10.03.93 Bulletin 93/10**

(84) Etats contractants désignés : **AT BE CH DE ES FR GB GR IT LI LU NL SE**

(56) Documents cités :
**EP-A- 0 242 416**
**CHEMICAL ABSTRACTS, vol. 103, no. 21, 25th November 1985, page 45, abstract no. 171707k, Columbus, Ohio, US; A.V. MAKSIMENKO et al.: "Water-soluble urokinase conjugates: fibrinolytic activity and other properties", & VOPR. MED. KHIM. 1985, 31(4), 12-20**

(56) Documents cités :
**EMBASE, no. 85201206; A.V. MAKSIMENKO et al.: "Water-soluble urokinase derivatives of combined action", & THROMB. RES. (USA), 1985, 38/3 (277-288) CODEN: THBRA**

(73) Titulaire : **ELF SANOFI**
**32-34, rue Marbeuf**
**F-75008 Paris (FR)**

(72) Inventeur : **Lormeau, Jean-Claude**
**34 rue du 8 mai**
**F-76150 Maromme (FR)**
Inventeur : **Choay, Jean**
**21 rue St-Guillaume**
**F-75007 Paris (FR)**
Inventeur : **Petitou, Maurice**
**Tour Mexico 65 rue du Javelot**
**F-75645 Paris Cedex 13 (FR)**

(74) Mandataire : **Polus, Camille et al**
**c/o Cabinet Lavoix 2, Place d'Estienne d'Orves**
**F-75441 Paris Cedex 09 (FR)**

EP 0 344 068 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des substances peptidiques modifiées par couplage covalent à des chaînes polyosidiques biocompatibles, ces dernières n'étant fixées à la substance peptidique que par leur extrémité réductrice. L'invention concerne également un procédé de préparation des substances peptidiques modifiées en question ainsi que les compositions pharmaceutiques les contenant et leur usage en thérapeutique.

Les polypeptides, surtout lorsqu'il s'agit des protéines et plus particulièrement des enzymes, sont des produits souvent peu stables dans l'organisme. Les préparations les contenant perdent de leur activité dans le temps et, lorsqu'elles sont administrées à l'homme en tant que médicament, elles sont rapidement catabolisées; leur demi-vie est ainsi de courte durée.

Différents moyens ont déjà été utilisés pour tenter de pallier ces inconvenients, et plus particulièrement le couplage covalent à des chaînes macromoléculaires. Parmi ces dernières, on a utilisé des chaînes aliphatiques linéaires hydroxylées, telles que le polyéthylène glycol ou le polypropylène glycol (PEG ou PPG) ou des polyosides de type dextran, les couplages s'effectuant soit entre les groupements hydroxylés et, selon les procédés, les groupements aminés ou carboxylés des protéines, soit entre les groupements carboxyles du polyoside et les groupements sulfhydriles ou amino du polypeptide (A.V. Maximenko et al, Vopr. Med. Khim. 1985, 31, 12-20, CA 1985, 103, 171707k).

Plus récemment, on a également proposé d'utiliser comme chaîne macromoléculaire un glycosaminoglycane particulier, la chondroitine sulfate, brevet européen 242 416. Les couplages proposés sont effectués par l'établissement de liaisons amides entre les restes des groupements carboxyles des chondroitines sulfates et les groupements amino des protéines.

Par ailleurs, il a été proposé de lier de façon covalente de l'hémoglobine à des fragments de glycosaminoglycane, brevet européen 0265865. Toutefois, dans ce cas, les glycosaminoglycanes ont été choisis parce qu'on connaissait leur capacité à former, avec l'hémoglobine, au moyen de leurs groupements sulfate et carboxyle, des liaisons ioniques qui modifient la conformation de cette dernière en vue d'augmenter sa capacité de rétention de l'oxygène. Il y a donc une interaction nécessaire entre le polypeptide et le polyoside, ce qui constitue un cas très particulier à l'opposé de l'objet de la présente invention.

Qu'il s'agisse de chaînes macromoléculaires de type PEG ou PPG, de polyosides de type dextran ou de chondroitines sulfates, il existe, pour chaque chaîne macromoléculaire dans les travaux décrits, plusieurs points de couplage, ce qui entraîne une modification de la structure tertiaire de la protéine couplée et une diminution de son activité.

On appelle structure tertiaire le repliement tri-dimensionnel de la chaîne d'acides aminés qui aboutit à une conformation native, stable et biologiquement active.

Il était donc intéressant de rechercher une méthode permettant de respecter davantage la structure tertiaire des polypeptides tout en leur conférant des caractéristiques pharmacologiques et pharmacocinétiques in vivo améliorées et une meilleure stabilité in vitro.

Selon la présente invention, il est possible d'effectuer un greffage de la chaîne polyosidique sur la chaîne peptidique en un point unique de ladite chaîne polyosidique en ayant recours à un groupe aldéhydique unique présent ou généré à l'extrémité de la chaîne polyosidique.

Il est bien connu que les sucres en général et les polysaccharides en particulier sont caractérisés par une structure hémiacétalique (cyclique ou fermée) en équilibre avec la structure aldéhydique correspondante (linéaire ou ouverte) selon le Schéma (A)

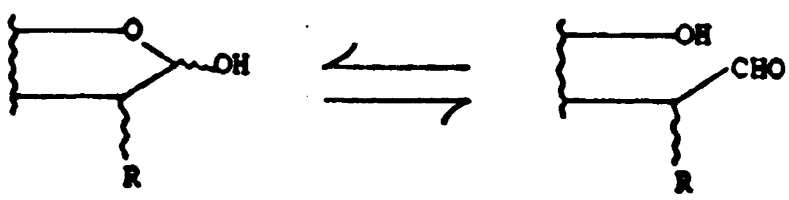

(A)

R étant par exemple un hydroxyle ou un groupe amino libre ou sulfaté, ledit équilibre étant, dans les sucres naturels, presque totalement déplacé vers la gauche.

Il est en outre connu que certaines méthodes de dépolymérisation de polysaccharides, notamment la dépolymérisation par action de l'acide nitreux, ci-après désignée "dépolymérisation nitreuse", conduisent à la transformation des unités osidiques. Ainsi, par exemple, les unités glycosidiques de l'héparine sont transformées, par la dépolymérisation nitreuse, en unités 2,5-anhydromannosidiques selon le Schéma (B)

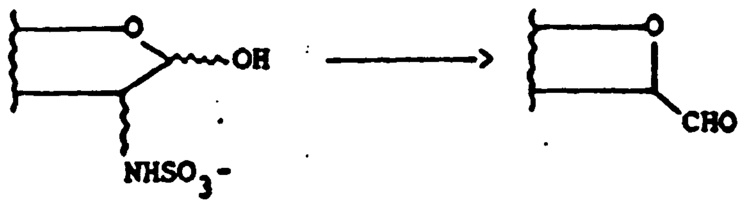

(B)

ladite unité 2,5-anhydromannosidique présentant un groupe aldéhydique.

La technique de dépolymérisation nitreuse a permis de préparer des héparines de bas poids moléculaire ayant à l'extrémité de la majorité des espèces le groupement 2,5-anhydro-mannose, donc un groupe aldéhydique.

On a maintenant trouvé qu'en faisant réagir un polysaccharide avec un polypeptide, la forme aldéhydique dudit polysaccharide réagit avec des amines libres dudit polypeptide pour donner une poly-base de Schiff. Cette réaction comporte le déplacement de l'équilibre du Schéma (A) ci-dessus vers la droite et la formation de la poly-base de Schiff.

On a aussi trouvé que la même réaction a lieu avec des polysaccharides obtenus par dépolymérisation nitreuse selon le Schéma (B) ci-dessus, et que la poly-base de Schiff se forme rapidement avec des rendements satisfaisants.

De plus, comme les polyosides n'ont qu'une seule extrémité réductrice, le couplage ne peut avoir lieu qu'au niveau de cette extrémité et la structure tertiaire du polypeptide est ainsi conservée.

On a encore trouvé que par réduction des poly-bases de Schiff on obtient des conjugués polysaccharide/polypeptide qui sont stables en milieu physiologique et qui confèrent au polypeptide une pharmacocinétique améliorée. Dans certains cas, ces conjugués possèdent à un dégré supérieur l'activité du polypeptide.

On a ultérieurement trouvé qu'en faisant réagir le polysaccharide avec le polypeptide en présence d'un agent réducteur, on obtient directement le conjugué polysaccharide/polypeptide sans isoler la poly-base de Schiff intermédiaire, avec de très bons rendements.

Ainsi, la présente invention concerne, selon un de ses aspects, un N-polyosyl-polypeptide de formule

$$(\text{Oside-Z}'\text{-CH}_2\text{-NH-})_n\text{P}' \qquad (I)$$

dans laquelle

- P′ est le radical n-valent d'un polypeptide P lié aux n groupes NH provenant de n groupes amino libres présents à l'origine dans la molécule de P, P′ étant autre que le radical monovalent de l'hémoglobine;
- Z′ est l'unité saccharidique terminale réductrice du polysaccharide Oside-Z dans sa forme aldéhydique, soit naturelle, soit provenant de la dépolymérisation nitreuse, privée du groupe aldéhydique de Z;
- Oside est le reste du polysaccharide Oside-Z;
- n est un nombre entier de 2 à 30.

ou, le cas échéant un de ses sels pharmaceutiquement acceptables.

Dans la formule I ci-dessus, P′ est avantageusement le radical n-valent d'un polypeptide P choisi parmi les hormones peptidiques glycosylées ou non, les toxines glycosylées ou non, les enzymes et les inhibiteurs d'enzyme.

Dans la présente description, le polypeptide, désigné "P", est schématiquement représenté par la formule

$$\text{P}^\circ(\text{NH}_2)\text{m} \qquad (II)$$

dans laquelle P° est le radical m-valent du polypeptide P auquel sont liés ses m groupes fonctionnels $NH_2$ libres, lesdits m groupes fonctionnels $NH_2$ étant le groupe amino terminal et les (m-1) groupes amino en position epsilon des lysines contenues dans le polypeptide.

Dans la formule (II) ci-dessus, m représente la totalité des groupes amino disponibles pour la formation des poly-bases de Schiff, mais le couplage n'a lieu que sur un certain nombre, désigné "n", de ces groupes amino; le radical n-valent lié aux groupes amino qui ont réagi avec le groupe aldéhydique de Z est ci-après désigné P′.

Il est entendu que, dans la présente description, le concept élargi de "valence" se réfère aux liaisons libres des radicaux ou des structures concernés. Ainsi, le radical P′ m-valent ou n-valent est le résidu du polypeptide P avec ses m ou n liaisons libres telles qu'illustrées ci-dessus et les structures bivalentes Ia″, Ib″, Ic″ et Id″ ci-dessous s'expliquent toutes seules.

Dans la présente description, le polysaccharide est schématiquement représenté par la formule

$$\text{Oside-Z} \qquad (III)$$

Z étant l'unité saccharidique terminale réductrice dudit polysaccharide dans sa forme aldéhydique soit naturelle, soit provenant d'une dépolymérisation nitreuse et Oside étant le reste de la chaîne dudit polysaccharide.

Le radical monovalent Oside-Z'- représente le polysaccharide Oside-Z tel que défini ci-dessus, privé du groupe aldéhydique de Z.

Plus particulièrement, dans la formule (I) Oside-représente le radical d'un polysaccharide Oside-Z (III), appelé également "polyoside", provenant de substances naturelles biocompatibles, sans résidus aglycones, ledit polyoside étant privé d'une unité saccharidique terminale réductrice (Z).

Le terme "biocompatible" tel qu'utilisé dans le présent descriptif désigne la propriété de polysaccharides naturels, ayant un poids moléculaire de préférence inférieur à 100.000, notamment compris entre 1.200 et 50.000, et de leurs dérivés ou analogues dont les constituants se trouvent naturellement dans l'organisme des mammifères et qui, de ce fait, peuvent être facilement métabolisés et éliminés.

De façon avantageuse, le polyoside provenant de substances naturelles biocompatibles est un glycosaminoglycane tel que défini par R. W. Jeanloz, Arthritis and Rheumatism, 1960, 3, 233-237 et par B. Casu, Advances in Carbohydrate Chemistry and Biochemistry, 1985, 43, 51-134.

De façon préférentielle, dans la formule I ci-dessus, Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

Dans un aspect particulier et avantageux de l'invention, le N-polyosyl-polypeptide provient d'un glycosaminoglycane du type héparinique Oside-Z dont le motif Z représente le motif terminal réducteur d'une chaîne d'héparine ayant subi une dépolymérisation nitreuse. Il est représenté par la formule (Ia) suivante:

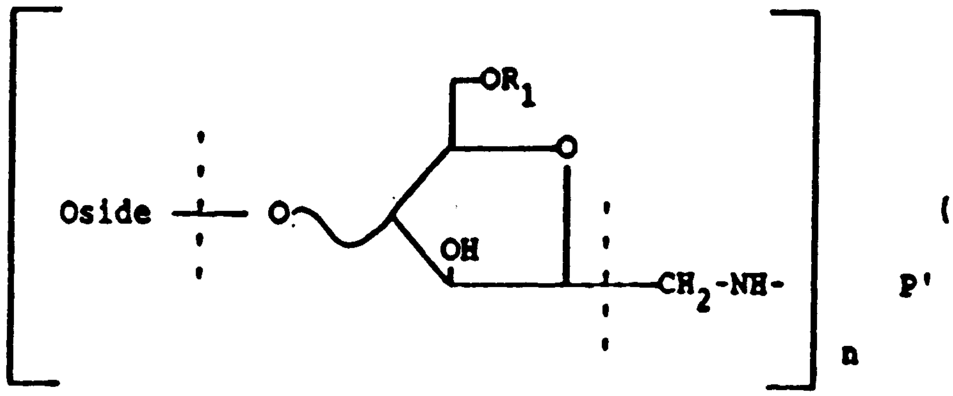

(Ia)

qui correspond à la formule (I), le groupement entre les lignes en pointillé étant le radical Z' et $R_1$ étant H ou $SO_3^-$.

Dans la formule (Ia) ci-dessus, Oside désigne, selon un aspect préférentiel de la présente invention, le radical provenant d'une héparine, représenté par la formule

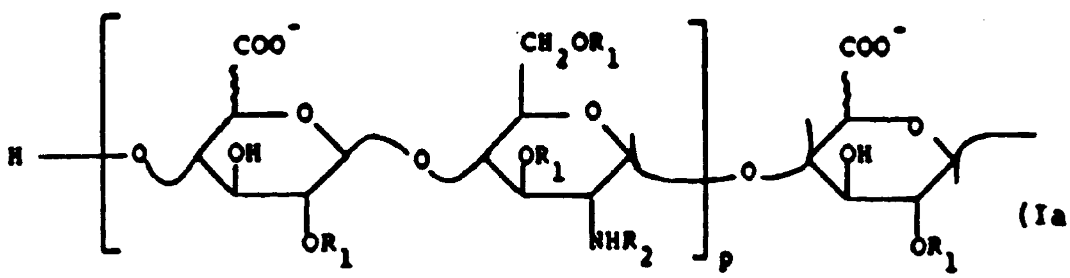

(Ia')

dans laquelle $R_1$ représente l'hydrogène ou un groupe $SO_3^-$, $R_2$ représente un groupe acétyle ou un groupe $SO_3^-$ et p est de 4 à 24.

Il est entendu que la valeur de p, dans le radical (Ia'), est telle qu'elle représente le nombre d'unités disaccharidiques de fragments d'héparine provenant d'une dépolymérisation nitreuse de l'héparine naturelle, dans des mélanges ayant avantageusement une masse moléculaire moyenne se situant entre 2000 et 8000 daltons, de préférence d'environ 4500 daltons ou d'environ 2500 daltons.

Dans un autre aspect particulier de l'invention, le N-polyosyl-polypeptide provient d'un polysaccharide Oside-Z dont le motif Z représente le motif terminal réducteur naturel d'un glycosaminoglycane de type héparinique. Il est représenté par la formule (Ib) suivante:

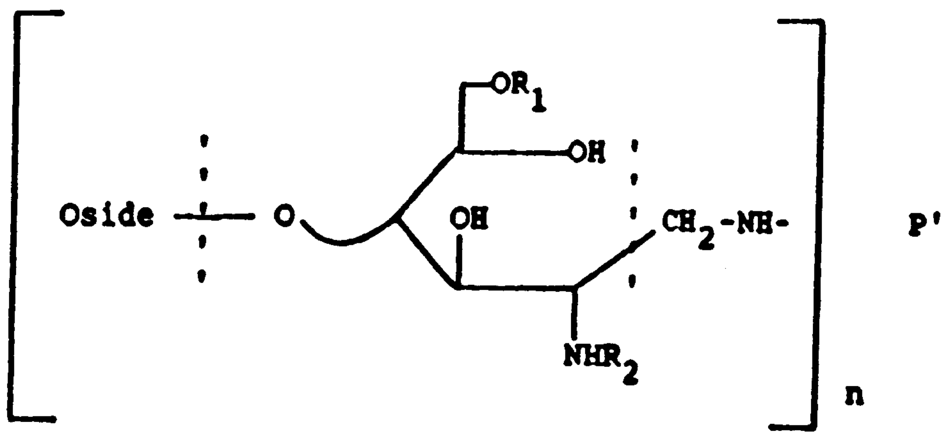

(Ib)

qui correspond à la formule (I), le groupement entre les lignes en pointillé étant le radical Z′, $R_1$ étant H ou $SO_3^-$, $R_2$ étant H, $SO_3^-$ ou acétyle et Oside étant le reste de la chaîne héparinique.

Dans la formule (Ib) ci-dessus, $R_2$ représente de préférence $SO_3^-$ ou acétyle et Oside représente de préférence le radical (Ia′), dans lequel p est de 10 à 100, la valeur de p étant telle qu'elle représente le nombre d'unités disaccharidiques qui forment les mélanges de chaînes présentes dans l'héparine naturelle.

Dans un autre aspect particulier de l'invention, le N-polyosyl-polypeptide provient d'un polysaccharide Oside-Z dont le motif Z représente le motif terminal réducteur naturel d'un glycosaminoglycane de type chondroitine ou dermatane sulfate. Il est représenté par la formule (Ic) suivante:

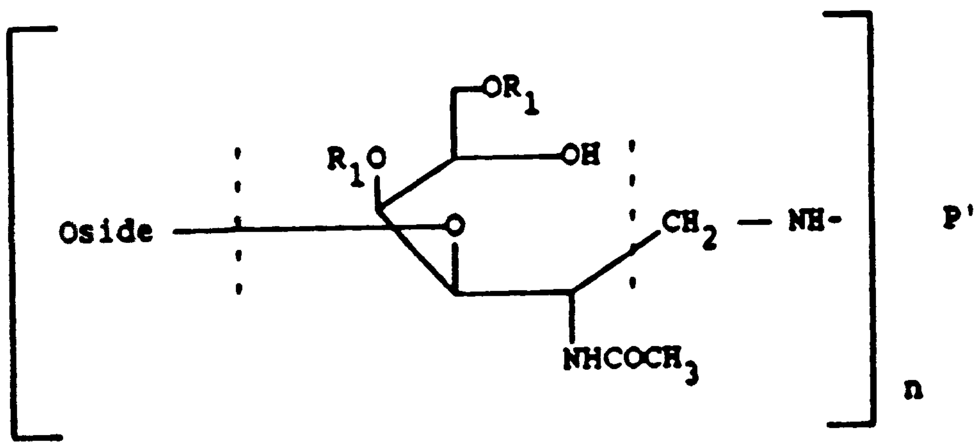

(Ic)

qui correspond à la formule (I), le groupement entre les lignes en pointillé étant le radical Z′, l'un des $R_1$ étant H et l'autre $SO_3^-$ et Oside étant le reste de la chaîne de la chondroitine sulfate ou du dermatane sulfate.

Dans la formule (Ic) ci-dessus, le radical Oside est représenté par la formule (Ic′)

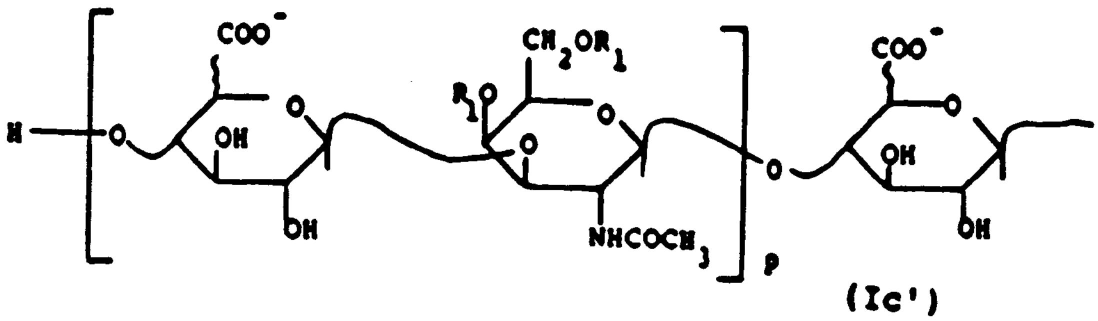

(Ic')

où l'un des $R_1$ représente l'hydrogène et l'autre un groupe $SO_3^-$ et p est de 10 à 100, la valeur de p étant telle qu'elle représente le nombre d'unités disaccharidiques qui forment les mélanges des chaînes présentes dans la chondroitine sulfate ou le dermatane sulfate.

Dans un autre aspect particulier de l'invention, le N-polyosyl-polypeptide provient d'un polysaccharide Oside-Z ayant subi une dépolymérisation dans des conditions telles que la chaîne possède un nombre de motifs impairs dont le motif Z réducteur représente un acide uronique, acide D-glucuronique ou L-iduronique. Il est alors représenté par la formule (Id) suivante:

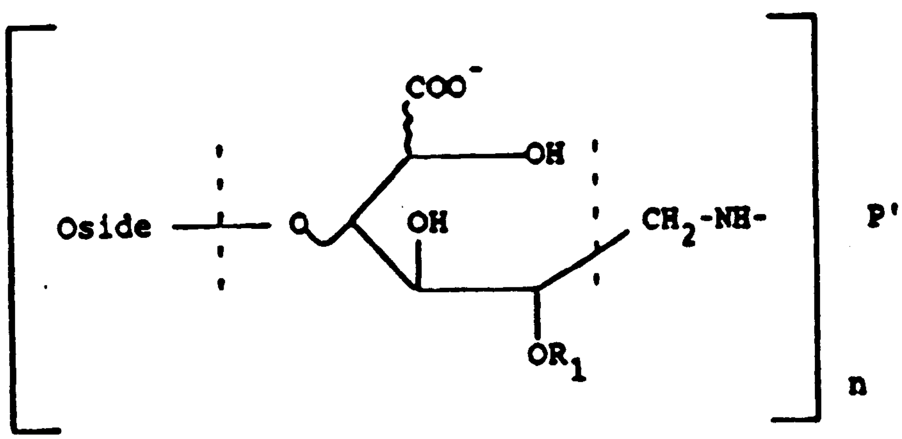

(Id)

qui correspond à la formule (I), le groupement entre les lignes en pointillé étant le radical Z', $R_1$ étant H ou $SO_3^-$, et Oside étant le reste de la chaîne polysaccharidique.

La présente invention comprend également des composés de formules (Ia) et (Ib) ci-dessus, dans lesquelles Oside représente le reste de fragments d'héparine homogènes au regard de leur masse moléculaire (formule Ia', p = 1-10) et contenant éventuellement le site de fixation à l'antithrombine III. Elle comprend en outre des composés de formule Ib ci-dessus, dans laquelle Oside représente le reste d'un oligosaccharide de synthèse (formule Ia', p = 1-6). Dans les formules (Ia), (Ib), (Ic) et (Id) ci-dessus, le radical Z' peut être mieux représenté par les structures bivalentes respectives suivantes:

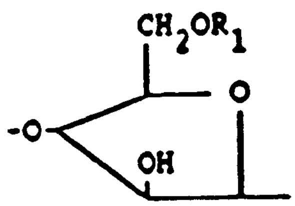

(Ia")

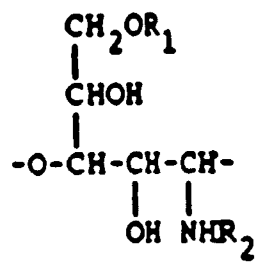

(Ib")

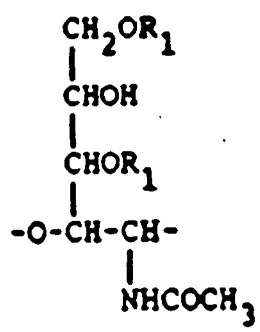

(Ic")

et

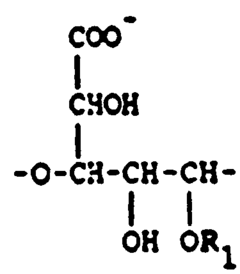

(Id")

dans lesquelles $R_1$ et $R_2$ sont tels que définis ci-dessus et la stéréoconfiguration est celle de l'unité terminale réductrice Z.

Comme indiqué plus haut, dans la formule I ci-dessus, P' est avantageusement le radical n-valent d'un polypeptide P choisi parmi les protéines, les hormones peptidiques glycosylées ou non, les toxines glycosylées ou non, les enzymes et les inhibiteurs d'enzymes, ledit polypeptide P étant naturel ou chimiquement modifié ou obtenu par recombinaison génétique.

Par exemple, P' peut être le radical n-valent d'une hormone peptidique telle qu'une hormone de croissance glycosylée ou non, notamment l'hormone de croissance humaine (hGH), le facteur libérant l'hormone de croissance (GRF), humain ou animal, naturel ou chimiquement modifié, ou un fragment actif, par exemple le GRF 1-44 ou le GRF 1-29, ou encore le facteur de croissance des cellules épithéliales (EGF) ou le facteur de croissance des fibroblastes (FGF) ou la somotostatine, ou encore une cytokine telle qu'une interleukine, de préférence l'interleukine 2 (IL-2), glycosylée ou non.

De même, P' peut être le radical n-valent d'une toxine, glycosylée ou non, notamment les protéines ou glycoprotéines inhibant les ribosomes, par exemple la ricine, la chaîne A de la ricine, la gélonine, la trichosantine ou encore la trichokinine.

P' peut également être le radical n-valent d'une enzyme thrombolytique tel que l'urokinase, la streptokinase, l'activateur tissulaire du plasminogène (tPA), la pro-urokinase, naturels, chimiquement modifiés ou obtenus par cominaison génétique, ou la superoxydedismutase.

P' peut aussi être le radical n-valent d'un polypeptide P inhibiteur d'enzymes, tel que l'hirudine, l'aprotinine, l'alpha-1-antitrypsine ou l'antithrombine III.

D'une manière avantageuse, le N-polyosyl-polypeptide de l'invention correspond à la formule (Ia) ci-dessus, dans laquelle P' est le résidu d'une molécule d'urokinase, et n est un nombre égal ou inférieur à 6, de préférence de 6,4 ou 3.

Dans un autre aspect avantageux de l'invention, P' est le radical d'une molécule de l'activateur tissulaire du plasminogène (tPA) et n est un nombre de 3 à 10, de préférence de 9, 8 ou 5.

Dans encore un autre aspect avantageux, P' est le résidu d'une molécule de streptokinase et n est inférieur ou égal à 4, de préférence de 2 à 4.

Il va de soi que P' peut être le radical n-valent de toute autre substance peptidique dès lors qu'elle possède les groupements amino libres permettant la liaison covalente avec le polyoside, P' étant autre que le radical monovalent de l'hémoglobine.

Selon un autre de ses aspects, la présente invention a pour objet un procédé de préparation du N-polyosyl-polypeptide de l'invention par greffage covalent de motifs amines libres d'une substance peptidique P sur des chaînes polyosidiques d'origine naturelle ne comportant chacune qu'un seul groupement aldéhydique susceptible de réagir avec un motif amine.

Plus particulièrement, la présente invention concerne un procédé pour la préparation d'un N-polyosyl-polypeptide de formule (I) ci-dessus caractérisé en ce qu'on traite un polypeptide P représenté par la formule

$$P°(NH_2)m \quad (II)$$

dans laquelle P° est le radical m-valent du polypeptide P auquel sont liés ses m groupes fonctionnels $NH_2$ libres, lesdits m groupes fonctionnels $NH_2$ étant le groupe amino terminal et les groupes amino en position epsilon des (m-1) lysines contenues dans le polypeptide, avec un excès d'un polyoside représenté par la formule

$$\text{Oside-Z} \quad (III)$$

Z étant l'unité saccharidique terminale réductrice dudit polysaccharide dans sa forme aldéhydique soit naturelle, soit provenant d'une dépolymérisation nitreuse et Oside étant le reste de la chaîne dudit polysaccharide et on soumet la poly-base de Schiff ainsi obtenue de formule

$$(\text{Oside-Z}'\text{-CH=N-})_nP' \quad (IV)$$

dans laquelle le radical monovalent Oside-Z'- représente le polysaccharide Oside-Z tel que défini ci-dessus, privé du groupe aldéhydique de Z, et P' et n sont tels que définis ci-dessus, à une réduction avec un cyanoborohydrure.

De façon avantageuse, les polyosides naturels biocompatibles Oside-Z utilisés comme produits de départ III sont des glycosaminoglycanes tels que définis ci-dessus (GAGs), notamment des substances constituées de chaînes dans lesquelles alternent des acides uroniques (acide D-glucuronique ou acide L-iduronique) et des sucres aminés, ces derniers pouvant être des glucosamines lorsqu'il s'agit de glycosaminoglycanes de type héparinique, ou des galactosamines lorsqu'il s'agit de glycosaminoglycanes de type chondroitine sulfate, à savoir la chondroitine sulfate 4-S, la chondroitine sulfate 6-S et le dermatane sulfate, les groupements carboxyles des motifs osidiques desdits GAGs pouvant être salifiés ou estérifiés, les groupes hydroxyles étant diversement substitués par des groupements fonctionnels, de préférence des groupes sulfates, et les groupements aminés étant substitués soit par des groupes sulfate, soit par des groupes acétyles.

De tels GAGs sont constitués d'un mélange de chaînes hétérogènes au regard de leurs poids moléculaires, ceux-ci pouvant varier de 1.800 à 50.000 daltons, et au regard de la position et du nombre de leurs groupements fonctionnels.

De préférence, le polyoside de départ III est un glycosaminoglycane choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

Des composés de départ III particulièrement préférés sont les glycosaminoglycanes de type héparinique, c'est-à-dire l'héparine, l'héparane sulfate et leurs dérivés obtenus soit par fractionnement à partir de l'héparine ou de l'héparane sulfate, soit par hémi-synthèse ou synthèse. De tels polyosides ont été largement décrits dans la littérature. Il peut s'agir de produits naturels obtenus par fractionnement ou synthèse, tels que ceux décrits dans les brevets français 2 440 376 et 2 461 719 ou dans EP 84 999 et 113 599; il peut s'agir également de produits obtenus par dépolymérisation tels que ceux décrits dans les brevets européens 37 319, 40 114 et 181 252, ou encore de produits sursulfatés comme ceux décrits dans le brevet européen 214 879. Dans un aspect particulièrement préféré de l'invention, on utilise des mélanges de fragments d'héparine obtenus par dépolymérisation nitreuse ayant une masse moléculaire inférieure à 10.000 daltons, en particulier soit ceux ayant une masse molécu-laire moyenne se situant entre 2.000 et 8.000 daltons, soit ceux ayant une masse moléculaire moyenne d'environ 4.500 daltons, soit encore ceux ayant une masse moléculaire moyenne d'environ 2.500 daltons. On peut également avantageusement utiliser des fragments d'héparine homogènes au regard de leur masse moléculaire ou des oligosaccharides obtenus par synthèse qui sont homogènes tant en ce qui concerne leur masse moléculaire que la position et le nombre de leurs groupes fonctionnels. Dans la suite du texte, le mélange de chaînes hépariniques obtenues par dépolymérisation nitreuse et ayant un poids moléculaire moyen de 4.500 daltons, préparé comme décrit dans l'exemple 1a ci-dessous, sera désigné par la référence "CY 216-CHO".

Un autre produit de départ avantageux Oside-Z est le mélange de fragments d'héparine obtenu par dépolymérisation nitreuse et décrit dans EP 37 319; il est désigné "CY 222-CHO".

On peut également choisir comme polyosides des dérivés de l'héparine dépourvus de site de fixation à l'antithrombine III (AT III), soit que les chaînes d'héparines aient été fractionnées de manière à éliminer les chaînes oligosaccharidiques comportant le site de fixation à l'AT III en ayant recours par exemple à une chromatographie d'affinité sur résine Sépharose-AT III ou à des chromatographies d'échange d'ions, comme il est décrit dans E. Sache et al., Thromb. Res., 1982, 25, 443-458, soit que ces sites aient été détruits par exemple par dépolymérisation à l'acide periodique et béta-élimination exhaustive. On rappellera ici que l'antithrombine III ou AT III est un inhibiteur plasmatique de la coagulation, actif sur différentes protéases plasmatiques et dont

l'activité est fortement accrue par l'héparine agissant comme co-facteur.

Il va de soi que les indications ci-dessus sont données uniquement à titre d'exemple, mais que tout polyoside naturel décrit dans la littérature et possédant les caractéristiques décrites peut être utilisé.

Les substances peptidiques P utilisées dans le procédé de la présente invention peuvent être soit des hormones peptidiques glycosylées ou non, soit des toxines glycosylées ou non, soit des enzymes ou des inhibiteurs d'enzymes naturels chimiquement modifiées ou obtenus par recombinaison génétique.

Dans un autre aspect avantageux de l'invention, les enzymes choisis sont des enzymes thrombolytiques.

D'une manière préférée, les enzymes thrombolytiques utilisés comme produits de départ sont l'urokinase, la streptokinase, l'activateur tissulaire du plasminogène, la pro-urokinase et leurs dérivés et analogues obtenus par recombinaison génétique ou par tout autre procédé.

D'une manière avantageuse, le couplage entre l'un des motifs aminés de la substance peptidique P et de la chaîne polyoside Oside-Z s'effectue au niveau du motif terminal de chaîne Oside-Z, cette dernière ayant ou non subi une opération préalable de fragmentation par dépolymérisation.

Selon la présente invention, il est possible de mettre en oeuvre directement une chaîne polyosidique possèdant un motif hémi-acétalique situé à son extrémité réductrice. Ce motif est en effet sous forme d'équilibre entre la forme cyclisée (fermée) et la forme linéaire (ouverte) et, au fur et à mesure de la consommation des groupements aldéhydiques par couplage de ces derniers aux groupements aminés de la substance peptidique P, de nouveaux groupements aldéhydiques sont libérés. Cette façon de procéder est avantageuse lorsque le polyoside ne possède pas de glucosamine N-sulfate sensible à l'acide nitreux ou lorsque l'on veut disposer de chaînes de masse moléculaire élevée. Néanmoins, elle présente l'inconvénient de nécessiter une durée de réaction beaucoup plus longue pouvant aller jusqu'à 10 à 15 jours. Il est donc d'une manière générale plus avantageux d'utiliser un polysaccharide Oside-Z dont le groupe aldéhydique a été généré par dépolymérisation selon une méthode appropriée, par exemple celle à l'acide nitreux.

Lorsqu'on utilise, comme produit de départ, un GAG Oside-Z dont le motif terminal Z est un groupe 2,5-anhydro-mannosique obtenu par dépolymérisation nitreuse selon le Schéma (B) ci-dessus, il est préférable que la réaction de couplage soit effectuée sur ledit Oside-Z immédiatement après sa préparation, pour éviter que le groupe aldéhydique, notoirement peu stable, perde sa réactivité.

Dans la mesure où l'on souhaite obtenir une chaîne polyosidique de type héparinique dépourvue de site de fixation à l'antithrombine III, il est avantageux d'utiliser comme produit de départ un GAG ayant subi une dépolymérisation à l'acide periodique, suivie d'une béta-élimination exhaustive, ce procédé permettant de couper préférentiellement la chaîne héparinique entre les carbones en positions 2 et 3 de tous les acides uroniques non sulfatés tel que le GAG décrit par Casu et al. Arzneim. Forsch./Drug Res., 1986, 36 (1), n. 4, 637-646. Or, on sait qu'un tel motif est présent dans le site de fixation de l'héparine à l'antithrombine III.

La quantité de chaînes polyosidiques à mettre en oeuvre doit être en fort excès par rapport aux chaînes peptidiques dans un rapport de 100 à 2000 moles de Oside-Z pour 1 mole de la substance peptidique P. Cet excès est avantageusement de 500 à 1500 moles d'Oside-Z pour 1 mole de la substance peptidique P. De préférence, lorsque l'on met en oeuvre de l'urokinase, on utilise 500 à 650 moles de Oside-Z pour 1 mole d'urokinase; et, lorsque l'on met en oeuvre l'activateur tissulaire du plasminogène, on utilise de préférence 600 à 1350 moles de Oside-Z pour 1 mole de l'enzyme.

Le milieu réactionnel est un milieu aqueux qui doit être dépourvu d'amines primaires ou secondaires susceptibles d'interférer avec la réaction de couplage entre Oside-Z et le polypeptide P. D'une manière avantageuse, le milieu aqueux est un tampon tel qu'un tampon phosphate ou bicarbonate ou tout autre tampon susceptible d'être ajusté au pH approprié par addition d'un acide minéral ou d'un hydroxyde alcalin. Eventuellement, il peut être additionné d'un solvant organique miscible à l'eau tel que l'éthanol, l'acétone, la diméthylformamide.

Dans un mode préféré de l'invention, on utilise un tampon phosphate 0,05 M additionné de chlorure de sodium. On y adjoint, si nécessaire, du polysorbate 80 (dérivés du sorbitane monooléate ou tween), par exemple à raison de 1 p. 10.000, ce surfactant permettant d'éviter l'absorption de la substance peptidique P sur les surfaces.

Le pH du milieu réactionnel est choisi de façon à ce que l'amine soit sous forme protonée, il peut varier de 6 à 9, il est toutefois avantageusement de 7 à 8, de préférence voisin de la neutralité.

La température de réaction peut varier en fonction de la substance peptidique utilisée, elle se situe avantageusement entre +2°C et +35°C pendant 2 à 15 jours. De préférence, on opère à température basse, par exemple à +4°C, sourtout lorsque la substance peptidique est un enzyme, un grand nombre de ces derniers étant thermolabiles, ce qui est en particulier le cas des enzymes thrombolytiques. Il faut noter toutefois que plus la température est basse, plus la cinétique de la réaction est lente. La durée de la réaction sera donc variable selon la température choisie. De façon avantageuse, on laisse la réaction s'effectuer sous agitation à +4°C pendant 2 à 8 jours si le polyoside utilisé possède un groupement aldéhydique provenant d'une dépolymérisation nitreuse. En particulier lorsque la substance peptidique mise en oeuvre est un enzyme thrombolytique tel que l'urokinase, la pro-urokinase ou l'activateur tissulaire du plasminogène, le maintien de la réaction à +4°C pendant une durée de 3 à 5 jours permet d'obtenir un rendement de greffage satisfaisant.

La poly-base de Schiff de formule (IV) ainsi obtenue peut être isolée selon les techniques connues, par exemple par précipitation avec un sel approprié, tel que le sulfate d'ammonium, et soumise à une réduction avec un cyanoborohydrure, tel que le cyanoboro-hydrure de sodium.

La réduction peut également être faite directement dans le milieu réactionnel, sans isoler la poly-base de Schiff.

Il est également possible de passer directement aux composés I par réaction du polypeptide P avec le polyoside Oside-Z en présence de cyanoborohydrure de sodium dans les conditions ci-dessus.

De cette façon, la poly-base de Schiff intermédiaire est réduite au fur et à mesure qu'elle se forme et le produit I est isolé en tant que seul produit final.

Pour séparer le conjugué ainsi obtenu des produits qui n'ont pas réagi, on peut opérer par précipitation, cette opération pouvant s'effectuer par exemple au moyen de sulfate d'ammonium que l'on additionne au milieu réactionnel en quantité suffisante pour saturer la solution, c'est-à-dire à raison d'environ 600 mg/ml. On laisse ensuite décanter et on centrifuge.

Cette opération peut être répétée après reprise du précipité en additionnant à nouveau celui-ci de sulfate d'ammonium dans les mêmes conditions.

On peut aussi avantageusement concentrer le conjugué obtenu sur une membrane d'ultrafiltration du type Amicon[R] YM 10 ou YM 30, la membrane étant choisie en fonction du poids moléculaire du conjugué.

Pour récupérer le N-polyosyl-polypeptide sous forme pure, on effectue une gel perméation à l'aide d'un gel choisi en fonction de la nature et de la taille du polypeptide. La gel filtration s'effectue de manière avantageuse sur une colonne contenant un gel de type ultrogel, par exemple celui vendu par la Société IBF (France) sous le nom d'Ultrogel[R] AcA 44 ou celle vendue par Pharmacia sous le nom de Sephacryl[R] S 200.

Dans un mode de réalisation avantageux, on dissout le précipité obtenu à l'étape précédente dans un tampon approprié à la substance peptidique P mise en oeuvre, on dépose ensuite la solution sur la colonne qui a été préalablement équilibrée à l'aide du même tampon. La gel filtration est suivie au moyen d'un spectrophotomètre à 280 nm et le pic de protéine est récupéré. Il contient la totalité du produit, dont on effectue ensuite la caractérisation et le dosage.

Les poly-bases de Schiff de formule (IV) ci-dessus sont des produits nouveaux qui représentent les intermédiaires clé dans la préparation des N-polyosyl-polypeptides de formule (I). Elles constituent donc un aspect ultérieur de la présente invention.

De façon préférentielle, dans la formule (IV) ci-dessus, Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

Particulièrement avantageuses sont les poly-bases de Schiff provenant d'un polysaccharide Oside-Z dont le motif Z représente le motif terminal réducteur d'une chaîne de glycosaminoglycane héparinique ayant subi une dépolymérisation nitreuse. Elle est représentée par la formule (IVa) suivante:

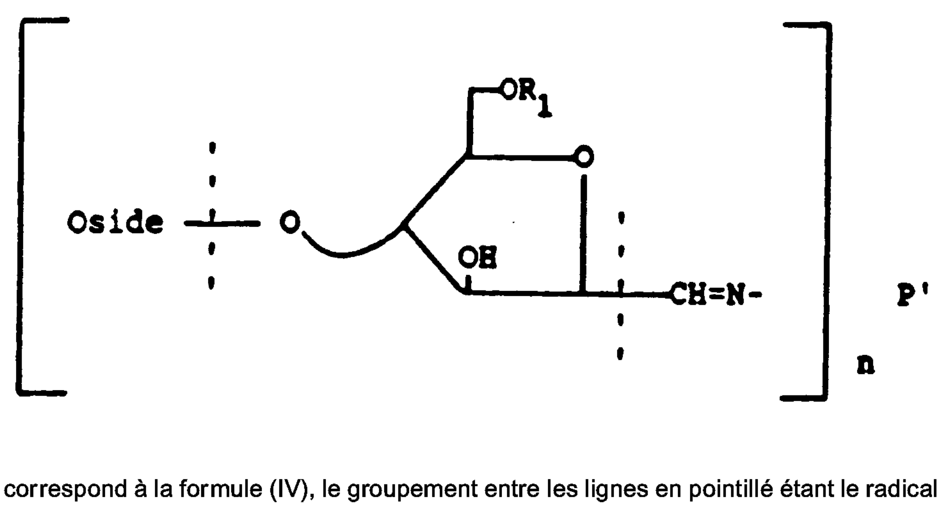

(IVa)

qui correspond à la formule (IV), le groupement entre les lignes en pointillé étant le radical Z' et $R_1$ étant H ou $SO_3^-$.

Dans un autre aspect particulier de l'invention, la poly-base de Schiff provient d'un polysaccharide Oside-Z dont le motif Z représente le motif terminal réducteur naturel d'un glycosaminoglycane de type héparinique. Elle est représentée par la formule (IVb) suivante:

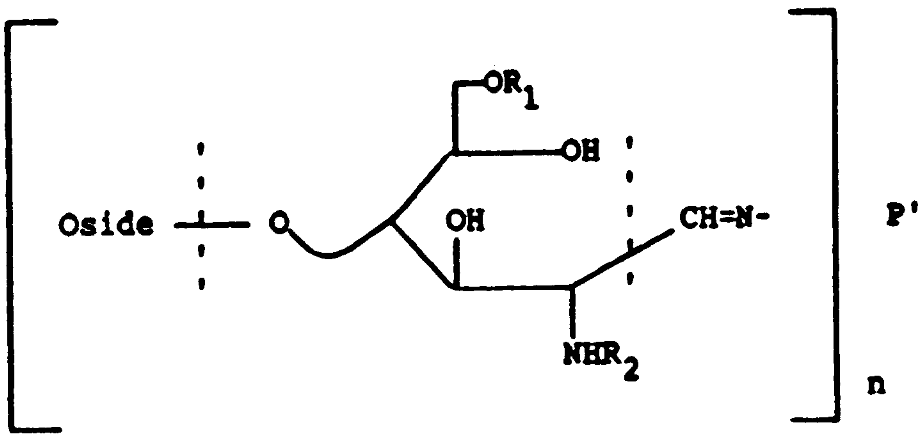

(IVb)

qui correspond à la formule (IV), le groupement entre les lignes en pointillé étant le radical Z', $R_1$ étant H ou $SO_3^-$, $R_2$ étant $SO_3^-$ ou Ac et Oside étant le reste de la chaîne héparinique.

Dans un autre aspect particulier de l'invention, la poly-base de Schiff provient d'un polysaccharide Oside-Z dont le motif Z représente le motif terminal réducteur naturel d'un glycosaminoglycane de type chondroitine sulfate. Elle est représentée par la formule (IVc) suivante:

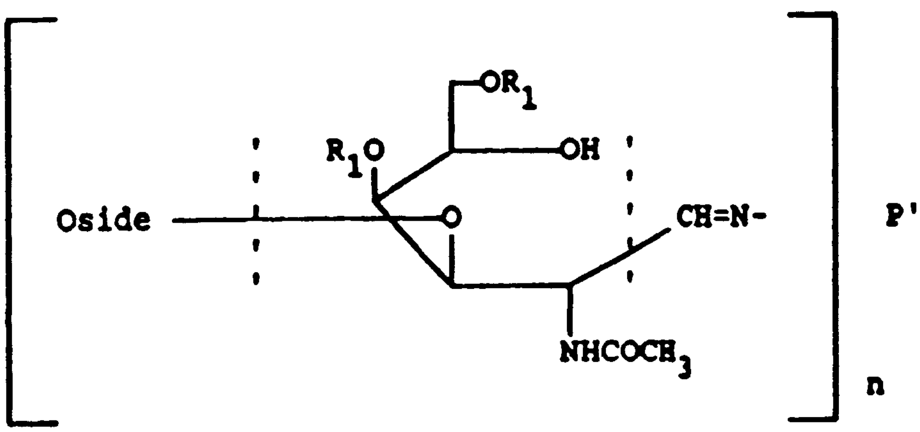

(IVc)

qui correspond à la formule (IV), le groupement entre les lignes en pointillé étant le radical Z', l'un des $R_1$ étant H et l'autre $SO_3^-$ et Oside étant le reste de la chaîne du type chondroitine sulfate.

Dans un autre aspect particulier de l'invention, la poly-base de Schiff provient d'un polysaccharide Oside-Z ayant subi une dépolymérisation dans des conditions telles que la chaîne possède un nombre de motifs impairs dont le motif Z réducteur représente un acide uronique, acide D-glucuronique ou L-iduronique. Elle est alors représentée par la formule (IVd) suivante:

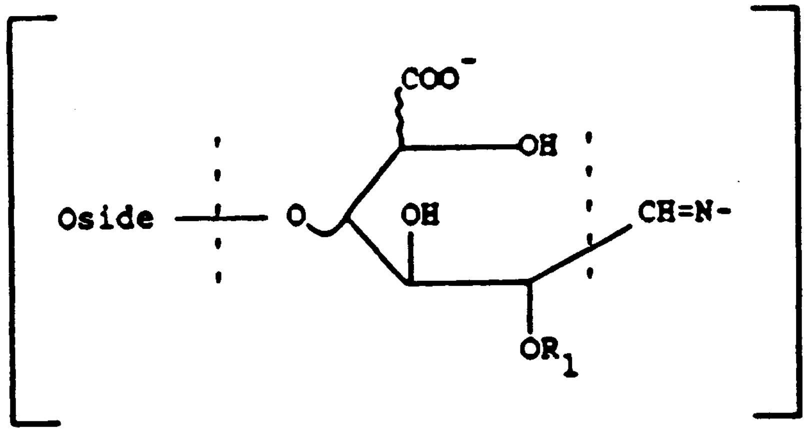

P' n (IVd)

qui correspond à la formule (IV), le groupement entre les lignes en pointillé étant le radical Z′, $R_1$ étant H ou $SO_3^-$, et Oside étant le reste de la chaîne polysaccharidique.

D'une manière avantageuse, la poly-base de Schiff de l'invention correspond à la formule (IVa) ci-dessus, dans laquelle P′ est une molécule d'urokinase, et n est un nombre égal ou inférieur à 6, de préférence de 6,4 ou 3.

Dans un autre exemple avantageux de l'invention, P′ est une molécule de l'activateur tissulaire du plasminogène (tPA) et n est égal ou inférieur à 10, de préférence 9, 8 ou 5.

Dans encore un autre exemple avantageux de l'invention, P′ est une molécule de streptokinase et n est un nombre inférieur ou égal à 4, de préférence de 2 à 4.

Les poly-bases de Schiff (IVa), (IVb), (IVc) et (IVd) ci-dessus sont transformées, selon la présente invention, en N-polyosyl-polypeptides correspondant (Ia), (Ib), (Ic) et (Id) par réduction avec le cyanoborohydrure de sodium.

L'invention a également pour objet l'utilisation des N-polyosyl-polypeptides de l'invention à titre de nouveaux médicaments, les indications thérapeutiques variant en fonction des activités particulières aux substances peptidiques utilisées pour la préparation du N-polyosyl-polypeptide. L'invention concerne en particulier, selon un aspect ultérieur, des compositions pharmaceutiques contenant à titre de substance active une quantité efficace d'au moins un quelconque des N-polyosyl-polypeptides de l'invention en association avec un véhicule pharmaceutiquement acceptable. Notamment, dans les compositions pharmaceutiques utilisables par voie parentérale, le véhicule pharmaceutique utilisé est un soluté chloruré ou glucosé. Les compositions pharmaceutiques en question sont utilisables par voie intraveineuse, intramusculaire ou sous-cutanée, ou dans des perfusions.

De telles compositions pharmaceutiques peuvent contenir de 5 à 100 mg par unité de prise. De façon avantageuse, lorsque le polypeptide utilisé est l'urokinase, le N-polyosyl-polypeptide utilisé dans la composition pharmaceutique est présent à raison de 0,2 à 2 mg par ml de préparation injectable. Lorsque le polypeptide utilisé est l'activateur tissulaire du plasminogène, le N-polyosyl-polypeptide utilisé dans la préparation injectable est présent à raison de 0,2 à 2 mg par ml de la préparation injectable.

Dans une autre réalisation de l'invention les compositions pharmaceutiques sont destinées à l'administration par voie orale et sont associées à des véhicules pharmaceutiques appropriés pour la préparation de gélules, tablettes, comprimés.

Dans une autre réalisations de l'invention, les compositions pharmaceutiques sont déstinées à des applications topiques et sprays, suppositoires, en association avec des excipients pharmaceutiques appropriés, il peut s'agir également de soluté isotonique aux larmes pour application oculaire, d'une manière générale sous toute forme pharmaceutique à la disposition du pharmacien.

L'invention concerne également à titre de réactif biologique nouveau les N-polyosyl-polypeptides de l'invention. En particulier, selon les substances peptidiques choisies, ils peuvent être utilisés comme réactif enzymatique ou comme produit de diagnostic ou à toute autre fin.

L'invention ne se limite pas aux aspects plus particuliers qui viennent d'être décrits, elle en embrasse au contraire toutes les variantes et elle sera mieux comprise à l'aide des exemples d'application qui suivent.

**EXEMPLE 1 : Préparation d'un N-polyosyl-polypeptide dans lequel P est l'urokinase et Z-oside est le CY 216-CHO.**

L'urokinase utilisée est préparée à partir d'urine humaine. Elle est constituée pour sa majeure partie d'urokinase de type $S_2$ d'un poids moléculaire de 54.000 daltons. Une telle urokinase existe dans le commerce. Son titre est de 85700 ul/mg selon la technique décrite par la Pharmacopée Européenne (Réf. 4ème Edition).

**Expérimentation n° 1:**

**(a) Génération de la terminaison aldéhydique par dépolymérisation de l'héparine:**

Le fragment d'héparine a été préparé de la manière suivante:

500 g d'héparine injectable, sous forme de sel de sodium, sont mis en solution dans 4500 ml d'eau déminéralisée, à une température de 18°C. Le rapport YW/USP de l'héparine utilisée est voisin de 1, ces titres présentant une valeur de l'ordre de 160-170.

La solution obtenue est mise sous agitation énergique, et son pH est abaissé à 2,5 par addition d'acide chlorhydrique concentré. On ajoute alors 15 g de nitrite de sodium dissous dans 300 ml d'eau. Le pH de la réaction est ajusté à 2,5 par de l'acide chlorhydrique concentré et le volume total de la solution est amené à 5000 ml. On laisse la réaction s'effectuer pendant 45 minutes puis on vérifie l'absence d'ions nitreux résiduels dans la solution réactionnelle, par exemple au moyen de papier indicateur imprégné d'iodure de potassium amidonné (développement d'une coloration bleue-violacée en présence d'ions $NO_2^-$.

On laisse la réaction se poursuivre jusqu'à disparition totale des ions nitreux en absence de réaction au papier iodo-amidonné, en effectuant des contrôles toutes les 3 ou 4 minutes.

Lorsque ces contrôles deviennent négatifs, la réaction est considérée comme étant achevée.

Les produits de la réaction sont récupérés par addition de 10 litres d'éthanol. Après 48 heures de repos, le produit est décanté et on élimine le surnageant.

Le précipité est redissous dans 9 litres d'eau déminéralisée.

On ajoute 100 g de chlorure de sodium, et on abaisse le pH de la solution à 3,8 à l'aide d'acide chlorhydrique concentré. Le volume est ajusté exactement à 10 litres à l'aide d'eau déminéralisée, et l'on ajoute sous agitation énergique 10 litres d'éthanol. On laisse reposer 48 heures. On siphonne le surnageant que l'on écarte. Le précipité est récupéré, lavé à l'éthanol, broyé, séché sous vide.

On obtient 230 grammes de produit CY 216-CHO ayant les caractéristiques suivantes:

| | |
|---|---|
| Titre USP | = 52 ul/mg |
| Titre Yin et Wessler | = 225 ul/mg |
| Poids moléculaire moyen | = 4000 à 5000 daltons |

**(b) Couplage de l'urokinase avec le CY 216-CHO:**

700 mg d'urokinase titrant 60.000.000 d'unités internationales sont mis en solution dans 900 ml de tampon phosphate 0.05M, NaCl 0.5M, pH 7. On ajoute 42 g de CY 216-CHO et 3 g de cyanoborohydrure de sodium ($NaBH_3CN$). On réajuste le pH à 7 avec de la soude 2N et on laisse réagir pendant 5 jours sous agitation, à la température de + 4°C.

**(c) Précipitation :**

On ajoute ensuite 600 mg/ml de sulfate d'ammonium $(NH_4)_2SO_4$ et on laisse décanter, puis on centrifuge à 15.000-20.000 rpm pendant 10 minutes. On récupère le culot que l'on redissout dans 50 ml du même tampon phosphate et l'on effectue une deuxième précipitation dans les mêmes conditions.

**(d) Gel filtration:**

Le produit récupéré à l'étape précedente est remis en solution dans le même tampon phosphate et est déposé sur une colonne Ultrogel$^R$ AcA 44 (Pharmacia, Suède) faisant 10 x 100 cm. L'opération est suivie au spectrophotomètre à 280 nm et on récupère le pic de protéine. Le pic correspond à 51.000.000 ul d'urokinase, soit un rendement en activité de 85%.

**(e) Caractérisation du produit:**

On étudie le taux de couplage du produit en dosant les protéines par la méthode au Bleu de Coomassie (S.M. Read et D.H. Northcote, Ann. Biochem., 1981, 116, 53-64) et le CY 216-CHO par la méthode au carbazole (dosage des acides uroniques, R. Bitter et H. Muir, Ann. Biochem., 1962, 4, 330-334).

Les résultats sont calculés pour un PM moyen de 4.500 daltons en ce qui concerne le CY 216-CHO et 54.000 daltons en ce qui concerne l'urokinase. On obtient 5 moles de CY 216-CHO pour 1 mole d'urokinase.

Le produit ainsi obtenu porte la référence IC 1857.

**Expérimentation n° 2:**

Différents essais ont été réalisés en faisant varier certains des paramètres opératoires:

1ére série d'experience pH basique:

-Variation de la durée de réaction:

| | |
|---|---|
| CY 216-CHO | 500 mg (111µM) |
| Urokinase | 20 mg (0,18µM) |
| NaCNBH3 | 50 mg |
| Tampon | phosphate K 0,2 M, pH 9 |
| Température | 4°C |

**Résultats:**

| Durée | Rapport molaire CY 216-CHO-UK dans le conjugué |
|---|---|
| 3 j. | 3/1 |
| 5 j. | 3/1 |
| 7 j. | 3/1 |

2éme série d'expérience pH neutre:

- Variation de température:

| | |
|---|---|
| CY 216-CHO | 1400 mg (311 µM) |
| Urokinase | 30 mg (0,54 µM) |
| $NaCNBH_3$ | 100 mg |
| Tampon | phosphate Na 0,05 M, pH 7, NaCl 0,5 M |
| Durée | 5 jours |

**Résultats:**

| Température | Rapport molaire CY 216-CHO-UK dans le conjugué |
|---|---|
| 4°C | 6/1 |
| 20°C | 6/1 |

Dans la première série d'expériences ci-dessus, on opère à un rapport molaire de 617, à un pH basique et à une température de + 4°C. Dans ces conditions, le taux de couplage est le même, c'est-à-dire 3 moles de CY 216-CHO pour 1 mole d'urokinase, et le rendement en activité enzymatique est de 100%.

Dans la deuxième série d'expériences ci-dessus où le rapport molaire et les quantités d'agent réducteur sont sensiblement les mêmes, mais où le pH utilisé est neutre, le résultat de couplage est supérieur à celui obtenu à un pH basique. Il est le même que l'on opère à + 4°C ou + 20°C. Toutefois, le rendement en activité enzymatique est supérieur à basse température, respectivement 85% au lieu de 60% de l'activité engagée.

**Expérimentation n° 3**

Un essai a été effectué en remplaçant l'étape (c) de précipitation au sulfate d'ammonium par une concentration de conjugué sur une membrane d'ultrafiltration sous pression d'azote.

Dans cet essai, l'urokinase utilisée a un titre de 70.000 ui/mg. Elle se trouve sous forme d'une solution concentrée que l'on soumet à une précipitation au sulfate d'ammonium afin de se trouver dans des conditions favorables pour le couplage.

**(a) Génération de la terminaison aldéhydique.**

Le fragment d'héparine CY 216-CHO a été préparé comme décrit plus haut (expérimentation n° 1).

**(b) Couplage de l'urokinase avec le CY 216-CHO**

1. Précipitation au sulfate d'ammonium de la solution concentrée d'urolinase

660 ml d'urokinase sont précipités à l'aide de sulfate d'ammonium $(NH_4)_2SO_4$ à raison de 550 g/l, le pH étant amené à 3,5 à l'aide d'$H_2SO_4$ dilué.

On laisse reposer une heure à 4°C et on centrifuge à 7.000 rpm pendant 30 minutes, puis à 15.000 rpm pendant 15 minutes.

Le précipité obtenu est dissous dans de l'eau distillée jusqu'à un volume de 80 ml auquel on ajoute 54 ml de tampon phosphate 0,05M, NaCl 0,5M, pH7.

2. Couplage avec le CY 216-CHO

On dilue 33 ml de la solution d'urokinase obtenue ci-dessus, soit 140 mg d'urokinase, jusqu'à un volume de 150 ml à l'aide du tampon utilisé plus haut. On ajoute sous agitation magnétique 7 g de CY 216-CHO et 0,5 g de $NaBH_3CN$. On ajuste le pH à 7 à l'aide de soude 2N et on laisse réagir pendant 5 jours sous agitation lente à +4°C.

**(c) Concentration sur membrane d'ultrafiltration**

Afin de séparer les produits ayant réagi de ceux n'ayant pas réagi, la solution obtenue à l'étape précédente est concentrée sur membrane Amicon$^R$ YM10 (Amico, France) sous pression d'azote.

**(d) Gel filtration**

Le produit récupéré à l'étape précédente est remis en solution dans le même tampon phosphate et est déposé sur une colonne Ultrogel$^R$ AcA 44 (Pharmacia, Suède) de 10 x 100 cm. L'opération est suivie au spectrophotomètre et on récupère le pic de protéine. Ce pic correspond à une activité spécifique de 60.000 ui/mg, soit un rendement en activité de 85%.

**(e) Caractérisation du produit**

Le taux de couplage du produit est calculé en dosant les protéines par la méthode au Bleu de Coomassie (ref. ci-dessus) et le CY 216-CHO par la méthode au carbazole (ref. ci-dessus).

Les résultats, calculés pour un PM moyen de 4.500 Daltons pour le CY 216-CHO et de 54.000 Daltons pour l'urokinase, donnent un rapport de 6 moles de CY 216-CHO pour une mole d'urokinase. Le produit ainsi obtenu porte la référence IC2026.

Il est lyophilisé après dilution dans de l'eau distillée à + 4°C pour obtenir une concentration en NaCl proche de 0,15M et addition de mannitol à raison de 10 mg /ml.

**EXEMPLE 2 : Préparation d'un N-polyosyl-polypeptide dans lequel P est le tPA et Z-oside est le CY 216-CHO.**

Le tPA utilisé possède un poids moléculaire de 60.000 daltons et titre 550.000 ul/mg. L'activité du produit est testée sur plaque de fibrine selon la technique de Ploug et Kjeldgaard (Biochim. Biophys. Acta, 1957, 24, 278-282).

**Expérimentation n° 1:**

**(a) Génération de la terminaison aldéhydique:**

Le fragment d'héparine CY 216-CHO a été préparé comme décrit à l'Exemple 1 ci-dessus.

**(b) Couplage du tPA avec le CY 216-CHO:**

2 mg de tPA sont mis en solution dans 3 ml de tampon phosphate 0.05 M, NaCl 1 M,tween 1 p. 10.000, pH 7. On ajoute 200 mg de CY 216-CHO et 8 mg de $NaCNBH_3$. On ajuste le pH à 7 avec quelques gouttes d'acide chlorhydrique 1N. On laisse réagir pendant 5 jours sous agitation, à la température de + 4°C.

A ce stade le rendement de couplage est de 90% (récupération de 990.000 ul). Ceci montre bien que la technique de couplage n'altère pas l'activité enzymatique.

**(c) Précipitation au sulfate d'ammonium:**

On sépare les produit qui ont réagi de ceux qui n'ont pas réagi par une précipitation au sulfate d'ammonium. On ajoute au milieu réactionnel 650 mg/ml de $(NH_4)_2SO_4$. On laisse décanter pendant 8 heures puis on centrifuge à 15.000-20.000 rpm pendant une demi-heure à + 4°C. Le précipité formé est récupéré.

On effectue ensuite une deuxième précipitation. Pour ce faire, on redissout le précipité précédemment formé récupéré dans 3 ml d'eau distillée et on ajoute à nouveau 650 mg/ml de $(NH_4)_2SO_4$ et on laisse à nouveau décanter 8 heures. On centrifuge à 18.000 rpm pendant une demi-heure à + 4°C.

Le précipité formé est récupéré et redissout dans 5 ml de tampon phosphate 0,05 M, NaCl 1M, Tween 1 p. 10.000, pH 7.

**(d) Caractérisation du produit:**

On prélève un aliquot de 0,2 ml de la solution précédemment obtenue. On effectue une gel filtration sur une colonne de Sephacryl$^R$ S 200 (Pharmacia, Suède) (100 cm x 2,5 cm) équilibrée avec de la soude 0,2 N, concentration nécessaire pour récupérer le tPA.

On récupère le pic de protéines et on dose les protéines par la méthode au Bleu de Coomassie (réf. ci-dessus) et le CY 216-CHO par la méthode au carbazole (dosage des acides uroniques, réf. ci-dessus).

Les résultats sont calculés pour un PM moyen de 4.500 daltons en ce qui concerne le tPA. On obtient 8 moles de CY 216-CHO pour 1 mole de tPA.

Le produit ainsi obtenu porte la référence IC 1903.

**Expérimentation n° 2:**

Un essai a été effectué en faisant varier le rapport molaire des produits mis en oeuvre: CY 216-CHO et tPA

tPA 2 mg (0,033 µM)
$NaCNBH_3$ 8 mg
Tampon phosphate Na 0,05 M, pH 7, NaCl 1 M, Tween 1 p. 10.000
Durée 5 jours
Température 4°C

| Résultats | Rapport molaire CY 216-CHO-tPA du conjugué |
|---|---|
| CY 216-CHO: 200 mg (44 µM) | 8/1 |
| CY 216-CHO: 100 mg (22 µM) | 5/1 |

On constate qu'avec un rapport molaire de 1346, on obtient un rendement de couplage de 8 moles de CY 216-CHO pour 1 mole de tPA, alors qu'avec un rapport de moitié, le rendement de couplage baisse notablement, 5 pour 1.

**Expérimentation n° 3**

Un essai a été effectué en remplaçant l'étape (c) de précipitation au sulfate d'ammonium par une concentration du conjugué sur une membrane d'ultrafiltration.

Le tPA utilisé est de l'Activase$^R$ (Genentech, USA), ayant un poids moléculaire de 75.000 daltons et un titre de 580.000 ui/mg. Ce tPA se trouvant conditionné avec une certaine quantité d'arginine, celle-ci est éliminée par une précipitation au sulfate d'ammonium pour permettre le couplage.

Une fois le conjugué obtenu, il est remis en solution dans un tampon contenant de l'arginine pour permettre sa lyophilisation dans des conditions favorables.

**(a) Génération de la terminaison aldéhydique**

Le fragment d'héparine CY 216-CHO a été préparé comme décrit plus haut à l'exemple 1.

**(b) Couplage du tPA avec le CY 216-CHO**

1. <u>Précipitation préalable du tPA au sulfate d'ammonium pour éliminer l'arginine</u>

Un flacon d'Activase$^R$ (Genentech, USA) est dissous dans 10 ml d'eau distillée.

5 ml de cette solution sont dilués dans 7,5 ml d'eau distillée. On ajoute du sulfate d'ammonium $(NH_4)_2SO_4$ à raison de 600 g/l et on laisse reposer 15 minutes à + 4°C. Après centrifugation à 18.000 rpm pendant 15 minutes à + 4°C, le culot est redissous dans 20 ml de tampon phosphate 0,05M; NaCl 1M, Tween 80, 1 p.10.000, pH7.

On répète 2 fois les opérations d'addition de sulfate d'ammonium et de centrifugation après dissolution du culot dans le tampon ci-dessus, les temps de repos étant respectivement de 30 minutes et d'une nuit, à + 4°C. Le dernier précipité est dissous dans 20 ml du tampon utilisé plus haut.

2. <u>Couplage avec le CY 216-CHO</u>

On ajoute à la solution ci-dessus 71 mg de $NaBH_3CN$ dissous dans 2 ml du même tampon et 1778 mg de CY 216 dissous dans 5 ml du même tampon. On ajuste le pH à 7 avec quelques gouttes de NaOH 2N.

On laisse réagir sous agitation douce pendant 4 jours à +4°C.

**(c) Concentration sur membrane d'ultrafiltration**

Afin de séparer les produits qui ont réagi de ceux qui n'ont pas réagi, on concentre la solution obtenue à l'étape b) à 5 ml sur membrane Amicon$^R$ YM30 (Amicon, France). Ces 5 ml sont dilués jusqu'à un volume de 50 ml par le tampon phosphate 0,05M, NaCl 1M, Tween 80 1 p. 10.000, pH7, puis à nouveau concentrée à 5 ml. Cette opération est répétée 3 fois. Le dernier concentrat est dilué à 20 ml à l'aide du tampon utilisé ci-dessus.

**(d) Caractérisation du produit**

On prélève 1 ml du concentrat dilué obtenu à l'étape (c). On effectue une gel filtration sur une colonne de Sephacryl$^R$ S200 (Pharmacia, Suède) (100 cm x 2,5 cm) équilibrée avec de la soude 0,2N. La fraction protéique est récupérée et on dose les protéines par la méthode au bleu de Coomassie (ref. ci-dessus).

Le CY 216-CHO est dosé par la méthode au carbazole (dosage des acides uroniques, ref. ci-dessus).

Les résultats calculés pour un PM moyen de 4.500 daltons pour le CY 216-CHO et de 75.000 daltons pour le tPA montrent un rapport molaire de 9/1.

Le produit ainsi obtenu porte la référence IC2025.

Il est lyophilisé après dilution dans un tampon contenant 1,7g de L-arginine, 0,5g d'acide phosphorique, 40 µl de Tween 80 à 10%, pH6.

<u>**EXEMPLE 3 : PREPARATION D'UN N-POLYOSYL-POLYPEPTIDE DANS LEQUEL P EST LA STREPTOKINASE ET Z-OSIDE EST LE CY 216-CHO.**</u>

La streptokinase utilisée, disponible dans le commerce a un poids moléculaire de 47.000 daltons et son titre est de 3.600 ui/mg.

Avant utilisation, elle a été purifiée par gel filtration. Après dissolution dans un tampon [$PO_4$-0,05M; NaCl 0,5M; pH7], on effectue une gel filtration sur colonne Ultrogel[R] ACA 44 (IBF, France) équilibrée avec le même tampon. Les fractions protéiques sont collectées puis concentrées sur membrane d'ultrafiltration Amicon YM10.

**(a) Génération de la terminaison aldéhydique.**

Le fragment CY 216-CHO a été préparé comme décrit plus haut à l'exemple 3.

**(b) Couplage de la streptokinase avec le CY 216-CHO**

Un flacon de streptokinase (250.000 ui/flacon) est dissous dans 2 ml de tampon phosphate ci-dessus. La solution est ensuite concentrée à 0,7 mg/ml, soit 0,14 μM.

On ajoute alors 2,78 mg de CY 216-CHO (soit environ 3,97 mg par mg de streptokinase) et 2,35 mg de $NaBH_3CN$ (soit 3,35 mg par mg de streptokinase).

Le pH est ajusté à 7 à l'aide de quelques gouttes de soude 2N, et on laisse réagir pendant 3 jours à +4°C sous agitation douce.

**(c) Gel filtration**

La solution obtenue à l'étape précédente est déposée sur une colonne Ultrogel[R] ACA 44 (IBF, France) faisant 5 x 100 cm, préalablement équilibrée en tampon phosphate utilisé plus haut. Le produit couplé est détecté au spectrophotomètre à 280 nm et récupéré.

**(d) Caractérisation du produit**

On dose les protéines par la méthode au bleu de Coomassie (ref. ci-dessus) et le CY 216-CHO par la méthode au carbazole (ref. ci-dessus).

Pour un PM moyen de 4.500 Daltons pour le CY 216-CHO et de 47.000 Daltons pour la streptokinase, on obtient un rapport de 2 moles de CY 216-CHO pour 1 mole de streptokinase.

Le produit ainsi obtenu porte la référence IC2024.

**EXEMPLE 4 : ACTIVITE PHARMACOLOGIQUE IN VITRO DES PRODUITS DE L'INVENTION**

**4.1. Activité enzymatique du produit IC 1857:**

L'activité urokinase du produit de couplage IC 1857 a été testée selon la technique de Ruyssen R. et Lauwers A. (Pharmaceuticals Enzymes, E. Story-Scientia, Gand, Belgium, 1978, 135-139) sur substrat synthétique AGLME. Le principe de la méthode consiste à doser au pH Staat l'hydrolyse de la N-alpha-acétyl-glycine-L-lysine méthyl ester.

Les résultats obtenus, comparés à ceux de l'urokinase de départ ont été les suivants:

| | |
|---|---|
| UK de départ | : 87.500 ul/mg |
| IC 1857 | : 74.000 ul/mg. |

**4.2 Activité enzymatique du produit IC 1903:**

L'activité tPA du produit de couplage IC 1903 est effectuée sur plaque de fibrine selon la technique de Ploug et Kjeldgaard comme indiquée plus haut. Le principe de la méthode consiste à déposer un aliquot de la solution à doser sur une boîte de Petri à la surface de laquelle on a formé un fil de fibrine contenant son propre plasminogène.

L'activité du produit à tester se traduit par des zones de lyse du fil de fibrine proportionnelles à la quantité de tPA déposée, par rapport à un étalon (étalon international de l'Organisation Mondiale de la Santé).

Les résultats obtenus, comparés à ceux du tPA de départ sont les suivants:

| | |
|---|---|
| tPA de départ | : 550.000 ul/mg |
| IC 1903 | : 302.000 ul/mg. |

**4.3 Activité enzymatique du produit IC2026**

L'activité urokinase du produit de couplage IC2026 a été testée :

1) sur substrat synthétique AGLME selon la technique de RUYSSEN ET Lauwers comme décrit au paragraphe 4.1,

2) sur plaque de fibrine selon la technique de Ploug et Kjelgaard comme décrit au paragraphe 4.2.

Les résultats obtenus pour l'urokinase de départ et pour le produit IC2026, après lyophilisation de ces 2 produits, sont les suivants :

| | AGLME | PLAQUE DE FIBRINE |
|---|---|---|
| UK de départ ui/mg | 62.500 | 53.000 |
| IC 2026 ui/mg | 67.000 | 200.300 |

Ces résultats montrent une activité fibrinolytique fortement accrue pour le produit couplé.

**4.4 Activité enzymatique du produit IC2025**

L'activité du produit de couplage IC2025 a été testée sur plaque de fibrine selon la technique de Ploug et Kjelgaard comme décrit au paragraphe 4.2.

Les résultats obtenus pour le tPA de départ et pour le produit IC2025, après lyophilisation des 2 produits, sont les suivants :

tPa de départ : 14 375 000 ui
IC 2025 : 11 300 000 ui

**4.5 Activité enzymatique du produit IC2024**

L'activité du produit IC2024 a été testée selon la technique du caillot. Dans cette méthode, on détermine la quantité de plasmine formée, provenant de la transformation du plasminogène en plasmine par la streptokinase, en mesurant le temps de lyse d'un caillot de fibrine. Ce temps de lyse est déterminé par rapport à une courbe étalon.

Le protocole utilisé est le suivant : on utilise du fibrinogène bovin en solution à 125 mg/25 ml de tampon phosphate 20mM, pH 7,2, de la thrombine (Roche, France) en solutionà 2 mg/5 ml du même tampon, di plasminogène (Kabi, Suède) en solution à 0,16 mg/ml de tampon et de la streptokinase (étalon interne) à 700 u/ml, diluée à 5 ui/ml.

On prépare une gamme étalon de 4 ui/ml à 1 ui/ml.

Pour le dosage, on incube pendant 10 minutes dans un bain- marie à 37°C 1 ml d'échantillon (étalon ou produit couplé), 0,1 ml de thrombine et 0,1 ml de plasminogène, auxquels on ajoute ensuite 1 ml de fibrinogène toutes les 5 secondes.

Pour calculer le titre, on trace la courbe du temps de lyse des caillots (en secondes) en fonction du nombre d'unités/ml de l'étalon. Le temps de lyse obtenu pour le produit couplé reporté sur la courbe permet de déduire le titre.

Les résultats obtenus pour la streptokinase de départ et pour le produit IC2024 sont les suivants :

Streptokinase de départ :70.000 u/mg
IC 2024 : 50.400 u/mg

## EXEMPLE 5 : ACTIVITE PHARMACOLOGIQUE IN VIVO DE IC 1857

### 5.1 Activité in vivo sur un modèle de détresse respiratoire provoquée, chez le hamster.

Le modèle utilisé consiste à mesurer, chez le hamster, le temps de détresse respiratoire provoquée par une embolie pulmonaire consécutive à une injection de thrombine.

Description du protocole:

Trois lots de cinq animaux sont utilisés:
- le 1er lot reçoit la thrombine seule,
- le 2ème lot reçoit la thrombine + UK (7.000 uI/kg),
- le 3ème lot reçoit la thrombine + IC 1857 (7.000 uI/kg).

Le produit à tester est injecté avant l'injection de la thrombine. Les produits sont injectés par voie intraveineuse à l'aide d'une pompe à perfusion (Palmer).
La détresse pulmonaire est observée et quantifiée par mesure de la durée de détresse, en distinguant la "détresse aigue" et la "détresse totale".
Le résultats figurant dans le tableau ci-après sont exprimés en pourcentage de réduction par rapport au témoin thrombine.

| Traitement | % de réduction de "détresse totale" | P | % de réduction de "détresse aigue" | P |
|---|---|---|---|---|
| Urokinase 7.000 uI/kg | 36,5 | < 0,02 | 36,4 | < 0,005* |
| IC 1857 7.000 uI/kg | 91,7 | < 0,001* | 93,3 | < 0,001* |
| * = fortement significatif | | | | |

En conclusion, on constate que la réduction de détresse respiratoire obtenue avec le conjugué IC 1857 est beaucoup plus importante que celle obtenue avec de l'urokinase seule, et ce, qu'il s'agisse de la détresse totale aussi bien que de la détresse aigue.

### 5.2 Activité thrombolytique

Etude de l'activité lytique de IC 1857

L'expérience réalisée a pour but d'étudier l'activité lytique d'un produit de l'invention, le conjugué d'urokinase IC 1857 avec celle de l'urokinase standard utilisé pour sa préparation.

Le protocole est le suivant :

L'étude est réalisée sur des animaux encagés une semaine.

Le modèle est caractérisé par l'induction d'une thrombose par injection d'un agent inducteur de thrombose, suivie d'une ligation temporaire des veines jugulaires de l'animal avec des pinces chirurgicales.

L'agent inducteur de thrombose est un complexe concentré de prothrombine (CCP) (25u/kg) et un mélange de venin de vipère Russel (VVR) (0,01 u/kg).

Le temps de circulation de l'agent induisant la thrombose est de 20 secondes.

Les deux veines jugulaires sont alors ligaturées, en commençant par la veine droite. La stase veineuse dure 10 minutes. Un écoulement partiel est initié en ouvrant les pinces de manière standard. Les produits de l'essai sont injectés par infusion standard avec une micropompe (300 secondes) d'une manière standard selon Agnelli (Thromb. Res. 1985, 40, 769-777). Les veines jugulaires sont ensuite incisées et les thrombi évalués immédiatement, en commençant par la veine droite.

Les thrombi sont évalués selon des critères numériques de 0 à 4:0 correspondant à l'absence de caillot et 4 à la présence d'un gros caillot unique. L'activité lytique est exprimée en pourcentage de lyse: 100% de lyse correspondant au degré 0 et 0% au degré 4.

Deux groupes de 15 lapins reçoivent l'un l'urokinase standard, l'autre, le conjugé IC 1857.

Le tableau suivant donne les résultats obtenus. Il existe une différence statistiquement significative (p < 0,0001) en faveur de IC 1857.

POURCENTAGE DE LYSE

| | | | | |
|---|---|---|---|---|
| 1 | 3,8 | 5,0 | 1,3 | 67,5 |
| 2 | 3,4 | 15,0 | 1,8 | 55,0 |
| 3 | 4,0 | 0 | 2,7 | 32,5 |
| 4 | 4,0 | 0 | 3,0 | 25,0 |
| 5 | 3,5 | 12,5 | 1,8 | 55,0 |
| 6 | 2,9 | 17,5 | 1,9 | 75,0 |
| 7 | 1,8 | 55,0 | 2,0 | 50,0 |
| 8 | 3,6 | 10,0 | 1,6 | 60,0 |
| 9 | 2,3 | 42,5 | 0 | 100,0 |
| 10 | 2,8 | 30,0 | 0 | 100,0 |
| 11 | 3,1 | 22,5 | 1,7 | 57,5 |
| 12 | 2,7 | 32,5 | 1,9 | 52,5 |
| 13 | 2,9 | 23,5 | 2,3 | 42,5 |
| 14 | 2,6 | 35,0 | 2,8 | 30,0 |
| 15 | 3,0 | 25,0 | 1,0 | 75,0 |
| x±1SD | 3,1±0,6 | 22,0±15,1 | 1,7±0,9 | 58,5±21,8 |

## EXEMPLE 6 : ETUDE PHARMACOCINETIQUE

### 6.1. Etude pharmacocinétique de IC 1903:

On a étudié comparativement la cinétique d'action du tPA natif et celle du produit préparé selon l'invention IC 1903 (Exemple 2, expérimentation 1). L'expérience a été effectuée chez le lapin (6 animaux par produit).

*Protocole d'étude:*

Après anesthésie, l'artère carotide gauche de chaque animal est cathétérisée. Un premier prélèvement de 2,5 ml de sang est pratiqué au temps 0 (T.0) sur citrate 3,8 % rapport au 1/10.

L'injection de 10.000 unités/kg du produit de référence et de IC 1903 est effectuée à la veine marginale de l'oreille droite, sous un volume moyen de 0,5 ml. Des prélèvements sont effectués à 1, 2, 3, 4, 5, 7, 10, 15, 20, 30, 45 et 60 minutes. Le sang total est centrifugé pour récupérer le plasma pauvre en plaquettes (PPP) et les euglobulines sont précipitées suivant une technique modifiée de N.A. Marsh et al. (Thromb. Haemost., 1977, 38, 545-546) et de J. Gram et al. (Thromb. Res., 1986, 49, 781-789). Les euglobulines sont ensuite déposées sur plaque de fibrine bovine et les aires de lyse sont mesurées après 18 heures à 37°C.

Résultats:

L'activité lytique observée sur plaque de fibrine est rapportée ci-dessous:

| Prélèvements | tPA de référence | IC 1903 |
|---|---|---|
| T.0 | 61,66 ± 35,48 | 68,66 ± 36,81 |
| T + 1 min. | 246,66 ± 66,49 | 355,83 ± 98,70 |
| T + 2 min. | 213,16 ± 50,55 | 301,50 ± 54,96 |
| T + 3 min. | 170,16 ± 49,11 | 272,00 ± 64,66 |
| T + 4 min. | 176,83 ± 75,00 | 259,33 ± 55,46 |
| T + 5 min. | 156,00 ± 40,61 | 247,50 ± 67,24 |
| T + 7 min. | 123,00 ± 35,60 | 218,83 ± 49,90 |
| T + 10 min. | 123,20 ± 29,52* | 197,66 ± 45,39 |
| T + 15 min. | 100,50 ± 28,14 | 160,33 ± 43,66 |
| T + 20 min. | 111,60 ± 18,74* | 142,00 ± 58,94* |
| T + 30 min. | 92,00 ± 46,00* | 131,66 ± 58,94** |
| T + 45 min. | 104,00 ± 53,75* | 110,75 ± 58,27*** |
| T + 60 min. | 121,60 ± 58,75* | 119,16 ± 43,73 |

N.B.: - surface de lyse en $mm^2$ : moyenne ± SD

* nombre d'animaux = 5

** nombre d'animaux = 3

*** nombre d'animaux = 4

**6.2. Etude pharmacocinétique de IC 1857:**

On a étudié comparativement la cinétique d'action de l'urokinase native et celle du produit préparé selon l'invention IC 1857 (Exemple 1, expérimentation 1). L'expérience a été effectuée chez le lapin (7 animaux par produit).

Protocole d'étude:

Les animaux sont anesthésiés comme dans l'exemple 6.1. ci-dessus et un premier prélèvement effectué au temps T.0.

Les animaux reçoivent ensuite 10.000 u/kg de produit de préférence et de IC 1857, dans les mêmes conditions que précedemment. Des prélèvements sont effectués à 1, 5, 10, 15, 20 et 30 minutes. Le sang total est centrifugé pour récupérer le plasma et les échantillons sont testés en tube à essai pour évaluation de leur activité amidolytique sur technique décrite par Kabi.

L'activité lytique est mesurée par densitométrie et transposée en unités Urokinase, selon une courbe d'étalonnage obtenue en passant dans les mêmes conditions opèratoires un étalon Urokinase.

Les résultats ont été les suivants:

| Prélèvements | UK de référence u/ml | IC 1857 u/ml |
|---|---|---|
| T.0 | 0,0 | 0,0 |
| T + 1 min. | 209,8 ± 40,7 | 85,8 ± 10,2** |
| T + 5 min. | 84,6 ± 25,7* | 51,2 ± 18,2 |
| T + 10 min. | 26,2 ± 15,4* | 34,8 ± 11,6 |
| T + 15 min. | 8,6 ± 7,0 | 17,8 ± 9,1** |
| T + 20 min. | 4,1 ± 4,8* | 16,5 ± 11,8 |
| T + 30 min. | 0,7 ± 1,3 | 5,2 ± 5,3 |

N.B.: * nombre d'animaux = 6

** nombre d'animaux = 5

On constate une pharmacocinétique allongée de IC 1857 par rapport à l'urokinase de référence, puisqu'aux temps + 15 min., + 20 min. et + 30 min., il y a encore une activité urokinasique dans le plasma des animaux.

## EXEMPLE 7 : ETUDE TOXICOLOGIQUE DES PRODUITS DE L'INVENTION

IC 1857 et IC 1903 ont été administrés en une injection unique à des souris, par voie intraveineuse, à la dose de 2 mg/kg. Les animaux ont été observés pendant 8 jours et n'ont présenté aucun signe de toxicité.

Comme il ressort de ce qui précède, les produits de l'invention présentent un très grand intérêt en tant que médicaments. En effet, la méthode de couplage choisie est particulièrement avantageuse. Le maintien d'une configuration spatiale appropriée de la substance peptidique couplée permet de conserver l'activité de la substance active et la conjugaison à un support polyosidique entraîne un renforcement de l'activité et un prolongement de celle-ci.

Les enzymes thrombolytiques tels que l'urokinase et l'activateur tissulaire du plasminogène conjugués au support polyosidique constituent donc un médicament thrombolytique très remarquable qui pourra être utilisé pour le traitement des thromboses et embolies veineuses et artérielles de formation récente, par exemple les thromboses coronaires, les embolies pulmonaires, les phlébites, l'infarctus du myocarde.

De manière avantageuse, IC 1857, produit de conjugaison de l'urokinase avec un fragment d'héparine pourra être administré à l'homme à raison de 1.000 à 10.000 ul/kg/heure, de préférence 2.000 à 4.000 ul/kg/heure, soit par perfusion intraveineuse continue ou injection intraveineuse lente, seul ou associé à un traitement de plasminogène.

## Revendications

### Revendications pour les Etats contractants suivants : AT, BE, CH, DE, FR, GB, GR, IT, LI, LU, NL, SE

**1.** N-polyosyl-polypeptide de formule

$$(Oside\text{-}Z'\text{-}CH_2\text{-}NH\text{-})_nP' \qquad (I)$$

dans laquelle

- P′ est le radical n-valent d'un polypeptide P lié aux n groupes NH provenant de n groupes amino libres présents à l'origine dans la molécule de P, P′ étant autre que le radical monovalent de l'hémoglobine;
- Z′ est l'unité saccharidique terminale réductrice du polysaccharide Oside-Z dans sa forme aldéhydique, soit naturelle, soit provenant de la dépolymérisation nitreuse, privée du groupe aldéhydique de Z;
- Oside est le reste du polysaccharide Oside-Z;
- n est un nombre entier de 2 à 30;

ou, le cas échéant un de ses sels pharmaceutiquement acceptables.

2. N-polyosyl-peptide selon la revendication 1 dans lequel P′ est le radical n-valent d'un polypeptide P choisi parmi les hormones peptidiques glycosylées ou non, les toxines glycosylées ou non, les enzymes et les inhibiteurs d'enzyme.

3. N-polyosyl-polypeptide selon la revendication 1 ou 2, caractérisé par la formule (I) ci-dessus, où Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

4. N-polyosyl-polypeptide selon l'une quelconque des revendications 1 à 3, caractérisé par la formule (I) dans laquelle P′ et n sont tels que définis dans la revendication 1;
   - Z′ est l'unité saccharidique terminale réductrice d'un glycosaminoglycane du type héparinique Oside-Z dans sa forme aldéhydique provenant de la dépolymérisation nitreuse de l'héparine, ledit Oside-Z étant constitué par un mélange de fragments d'héparine ayant une masse moléculaire moyenne se situant entre 2000 et 8000 daltons;
   - Oside est le reste du polysaccharide Oside-Z.

5. N-polyosyl-polypeptide selon l'une quelconque des revendications 1 à 4,caractérisé par la formule (I) dans laquelle P′ et n sont tels que définis dans la revendication 1;
   - Z′ est l'unité saccharidique terminale réductrice d'un glycosaminoglycane du type héparinique Oside-Z dans sa forme aldéhydique provenant de la dépolymérisation nitreuse de l'héparine, ledit Oside-Z étant constitué par un mélange de fragments d'héparine ayant une masse moléculaire moyenne d'environ 4500 daltons;
   - Oside est le reste du polysaccharide Oside-Z.

6. N-polyosyl-polypeptide selon l'une quelconque des revendications 1 à 5 caractérisé par la formule (I) dans laquelle Z′ a la structure

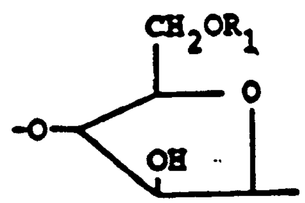

où $R_1$ est l'hydrogène ou un groupe $SO_3^-$.

7. N-polyosyl-polypeptide selon l'une quelconque des revendications 1 à 6, caractérisé par la formule (I) dans laquelle
   - P′ est le radical n-valent d'un enzyme thrombolytique lié aux n groupes NH provenant de n groupes amino libres présents à l'origine dans la molécule dudit enzyme thrombolytique;
   - n est de 2 à 10.

8. N-polyosyl-polypeptide selon la revendication 7, caractérisé par la formule (I) dans laquelle
   - P′ est le radical n-valent de l'urokinase lié aux n groupes NH provenant de n groupes amino libres présents à l'origine dans la molécule de ladite urokinase;
   - n est égal ou inférieur à 6, de préférence de 6,4 ou 3.

9. N-polyosyl-polypeptide selon la revendication 7, caractérisé par la formule (I) dans laquelle
   - P′ est le radical n-valent de la streptokinase lié aux n groupes NH provenant de n groupes amino libres présents à l'origine dans la molécule de ladite streptokinase,
   - n est de 2 à 4.

10. N-polyosyl-polypeptide selon la revendication 7, caractérisé par la formule (I) dans laquelle
   - P′ est le radical n-valent du tPA lié aux n groupes NH provenant de n groupes amino libres présents à l'origine dans la molécule dudit tPA;
   - n est de 3 à 10.

**11.** N-polyosyl-polypeptide de formule

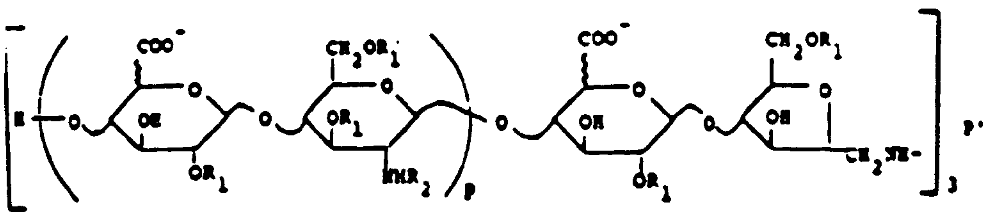

dans laquelle

- $R_1$ est l'hydrogène ou un groupe $SO_3^-$,
- $R_2$ est un groupe acétyle ou un groupe $SO_3^-$,
- P' est le radical trivalent de l'urokinase lié aux 3 groupes NH provenant de 3 groupes amino libres présents à l'origine dans la molécule de l'urokinase, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**12.** N-polyosyl-polypeptide de formule

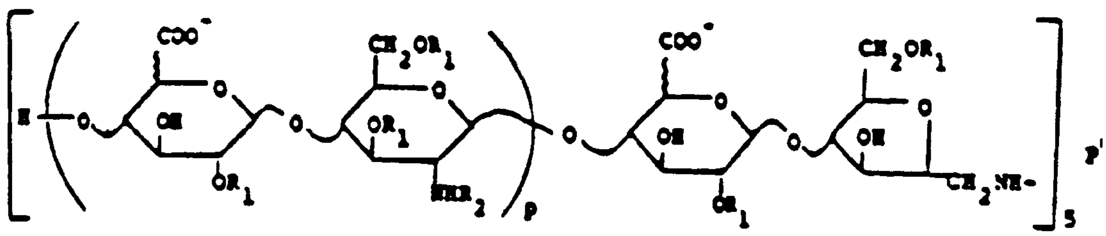

dans laquelle

- $R_1$ est l'hydrogène ou un groupe $SO_3^-$,
- $R_2$ est un groupe acétyle ou un groupe $SO_3^-$,
- P' est le radical pentavalent du tPA lié aux 5 groupes NH provenant de 5 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire d'environ 4500 daltons et contenant le site de fixation à l'AT III.

**13.** N-polyosyl-polypeptide de formule

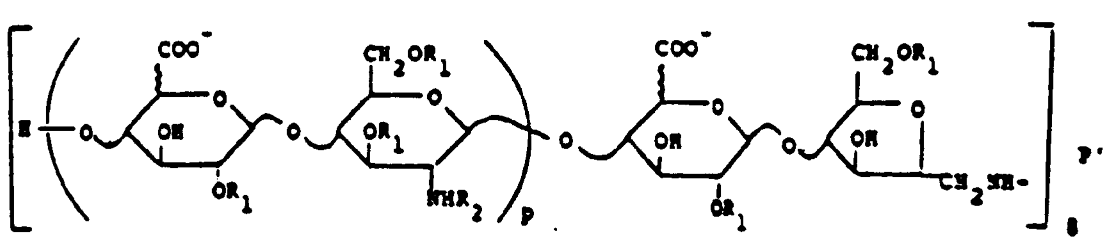

dans laquelle

- $R_1$ est l'hydrogène ou un groupe $SO_3^-$,
- $R_2$ est un groupe acétyle ou un groupe $SO_3^-$,
- P' est le radical octovalent du tPA lié aux 8 groupes NH provenant de 8 groupes amino libres présents à l'origine dans la molécule du tPA, et
- p, de 4 à 24, représente le nombre d'unités disaccharidiques de fragments d'héparine provenant d'une

dépolymérisation nitreuse dans un mélange ayant une masse moléculaire d'environ 4500 daltons et contenant le site de fixation à l'AT III.

**14.** N-polyosyl-polypeptide de formule

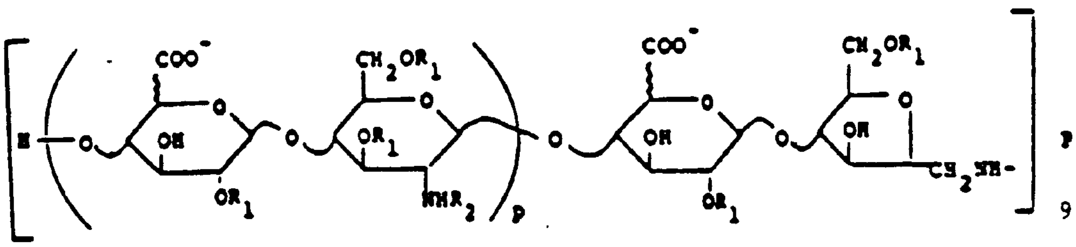

dans laquelle

- $R_1$ est l'hydrogène ou un groupe $SO_3^-$,
- $R_2$ est un groupe acétyle ou un groupe $SO_3^-$,
- P′ est le radical nonavalent du tPA lié aux 9 groupes NH provenant de 9 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire d'environ 4500 daltons et contenant le site de fixation à l'AT III.

**15.** Procédé pour la préparation d'un N-polyosyl-polypeptide selon la revendication 1, caractérisé en ce qu'on traite un polypeptide P représenté par la formule

$$P°(NH_2)m \qquad (II)$$

dans laquelle P° est le radical m-valent du polypeptide P auquel sont liés ses m groupes fonctionnels $NH_2$ libres, lesdits m groupes fonctionnels $NH_2$ étant le groupe amino terminal et les groupes amino en position epsilon des (m-1) lysines contenues dans le polypeptide, avec un excès d'un polyoside représenté par la formule

$$\text{Oside-Z} \qquad (III)$$

Z étant l'unité saccharidique terminale réductrice dudit polysaccharide dans sa forme aldéhydique soit naturelle, soit provenant d'une dépolymérisation nitreuse et Oside étant le reste de la chaîne dudit polysaccharide et on soumet la poly-base de Schiff ainsi obtenue de formule

$$(\text{Oside-Z}'\text{-CH=N-})_nP' \qquad (IV)$$

dans laquelle le radical monovalent Oside-Z′- représente le polysaccharide Oside-Z tel que défini ci-dessus, privé du groupe aldéhydique de Z, et P′ et n sont tels que définis dans la revendication 1, à une réduction avec un cyanoborohydrure.

**16.** Procédé selon la revendication 15, caractérisé en ce que, comme polyoside Oside-Z de départ, on utilise un mélange de fragments d'héparine obtenus par dépolymérisation nitreuse ayant une masse moléculaire moyenne se situant entre 2000 et 8000 daltons.

**17.** Procédé selon l'une quelconque des revendications 15 et 16, caractérisé en ce que, comme polyoside Oside-Z de départ, on utilise un mélange de fragments d'héparine obtenus par dépolymérisation nitreuse ayant une masse moléculaire moyenne d'environ 4500 daltons.

**18.** Procédé selon l'une quelconque des revendications 15 à 17, caractérisé en ce que, comme cyanoborohydrure, on utilise le cyanoborohydrure de sodium.

**19.** Procédé selon l'une quelconque des revendications 15 à 18, caractérisé en ce que, comme cyanoborohydrure, on utilise le cyano-borohydrure de sodium, la réaction entre $P°(NH_2)m$ et Oside-Z étant conduite en présence dudit cyanoborohydrure de sodium de façon à obtenir directement le N-polyosyl-polypeptide final.

**20.** Poly-base de Schiff de formule

$$(\text{Oside-Z}'\text{-CH=N-})_nP' \qquad (IV)$$

dans laquelle

- P′ est le radical n-valent d'un polypeptide P lié aux n groupes NH provenant de n groupes amino libres présents à l'origine dans la molécule de P, P′ étant autre que le radical monovalent de l'hémoglobine.
- Z′ est l'unité saccharidique terminale réductrice du polysaccharide Oside-Z dans sa forme aldéhydique, soit naturelle, soit provenant de la dépolymérisation nitreuse, privée du groupe aldéhydique de Z;
- Oside est le reste du polysaccharide Oside-Z;
- n est un nombre entier de 2 à 30.

**21.** Poly-base de Schiff selon la revendication 20, caractérisée en ce que P′ est le radical n-valent d'un polypeptide P choisi parmi les hormones peptidiques glycosylées ou non, les toxines glycosylées ou non, les enzymes et les inhibiteurs d'enzyme.

**22.** Poly-base de Schiff selon la revendication 20 ou 21, caractérisée par la formule IV ci-dessus, où Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

**23.** Poly-base de Schiff selon la revendication 20, caractérisée par la formule IV dans laquelle
- Z′ est l'unité saccharidique terminale réductrice d'un glycosaminoglycane du type héparinique Oside-Z dans sa forme aldéhydique provenant de la dépolymérisation nitreuse de l'héparine, ledit Oside-Z étant constitué par un mélange de fragments d'héparine ayant une masse moléculaire moyenne se situant entre 2000 et 8000 daltons;
- Oside est le reste du polysaccharide Oside-Z.

**24.** Poly-base de Schiff selon la revendication 20, caractérisée par la formule IV dans laquelle P′ et n sont tels que définis dans la revendication 20;
- Z′ est l'unité saccharidique terminale réductrice d'un glycosaminoglycane du type héparinique Oside-Z dans sa forme aldéhydique provenant de la dépolymérisation nitreuse de l'héparine, ledit Oside-Z étant constitué par un mélange de fragments d'héparine ayant une masse moléculaire moyenne d'environ 4500 daltons;
- Oside est le reste du polysaccharide Oside-Z.

**25.** Poly-base de Schiff selon l'une quelconque des revendications 20 à 24, caractérisée par la formule IV dans laquelle Z′ a la structure

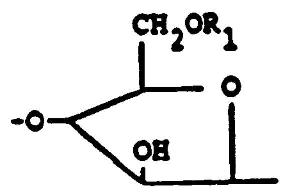

dans laquelle $R_1$ est l'hydrogène ou un groupe $SO_3^-$.

**26.** Poly-base de Schiff selon l'une quelconque des revendications 20 à 25, caractérisé par la formule (IV) dans laquelle
- P′ est le radical n-valent d'un enzyme thrombolytique lié aux n groupes NH provenant de n groupes amino libres présents à l'origine dans la molécule dudit enzyme thrombolytique;
- n est de 2 à 10.

**27.** Poly-base de Schiff selon la revendication 26, caractérisé par la formule (IV) dans laquelle
- P′ est le radical n-valent de l'urokinase lié aux n groupes NH provenant de n groupes amino libres présents à l'origine dans la molécule de ladite urokinase;
- n est égal ou inférieur à 6, de préférence de 6,4 ou 3.

**28.** Poly-base de Schiff selon la revendication 26, caractérisé par la formule (IV) dans laquelle
- P′ est le radical n-valent de la streptokinase lié aux n groupes NH provenant de n groupes amino libres présents à l'origine dans la molécule de ladite streptokinase,

- n est de 2 à 4.

**29.** Poly-base de Schiff selon la revendication 26, caractérisé par la formule (IV) dans laquelle
- P' est le radical n-valent du tPA lié aux n groupes NH provenant de n groupes amino libres présents à l'origine dans la molécule dudit tPA;
- n est de 3 à 10.

**30.** Poly-base de Schiff de formule

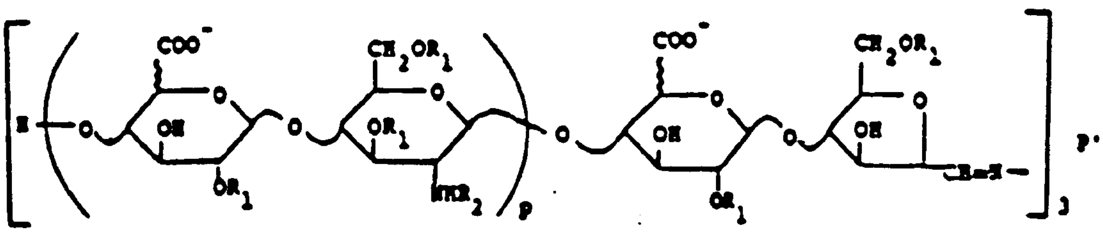

dans laquelle
- $R_1$ est l'hydrogène ou un groupe $SO_3^-$,
- $R_2$ est un groupe acétyle ou un groupe $SO_3^-$,
- P' est le radical trivalent de l'urokinase lié aux 3 groupes NH provenant de 3 groupes amino libres présents à l'origine dans la molécule de l'urokinase, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**31.** Poly-base de Schiff de formule

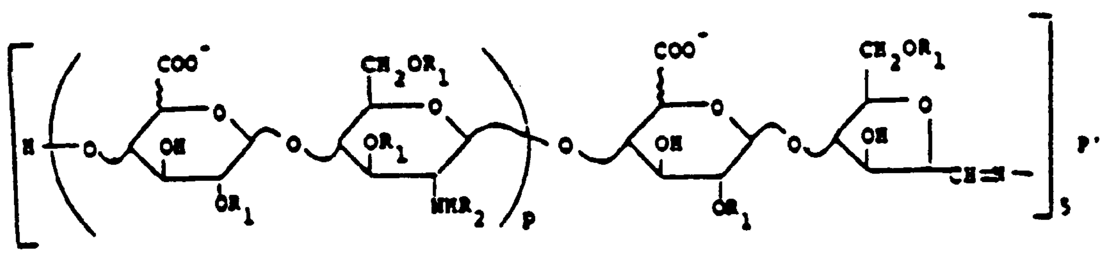

dans laquelle
- $R_1$ est l'hydrogène ou un groupe $SO_3^-$,
- $R_2$ est un groupe acétyle ou un groupe $SO_3^-$,
- P' est le radical pentavalent du tPA lié aux 5 groupes NH provenant de 5 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**32.** Poly-base de Schiff de formule

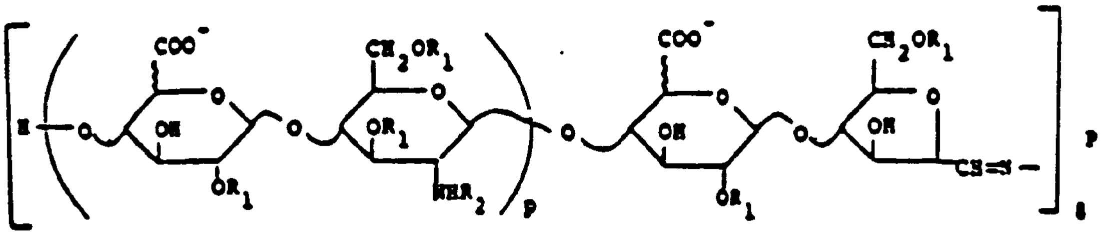

dans laquelle

- $R_1$ est l'hydrogène ou un groupe $SO_3^-$,
- $R_2$ est un groupe acétyle ou un groupe $SO_3^-$,
- P′ est le radical octovalent du tPA lié aux 8 groupes NH provenant de 8 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**33.** Poly-base de Schiff de formule

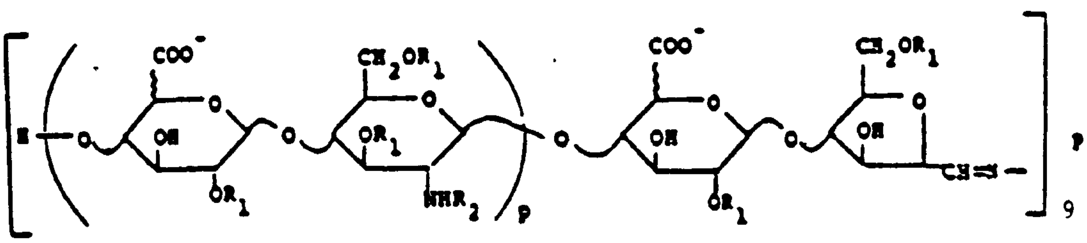

dans laquelle

- $R_1$ est l'hydrogène ou un groupe $SO_3^-$,
- $R_2$ est un groupe acétyle ou un groupe $SO_3^-$,
- P′ est le radical monovalent du tPA lié aux 9 groupes NH provenant de 9 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**34.** Composition pharmaceutique caractérisée en ce qu'elle renferme à titre de substance active une quantité efficace d'au moins un N-polyosyl-polypeptide selon l'une quelconque des revendications 1 à 14 en association avec un véhicule pharmaceutique.

**35.** Réactif biologique constitué par un produit selon l'une quelconque des revendications 1 à 14.

**Revendications pour l'Etat contractant suivant : ES**

**1.** Procédé de préparation de N-polyosyl-polypeptide de formule

$$(Oside\text{-}Z'\text{-}CH_2\text{-}NH\text{-})_nP' \qquad (I)$$

dans laquelle

- P′ est le radical n-valent d'un polypeptide P lié aux n groupes NH provenant de n groupes amino libres présents à l'origine dans la molécule de P, P′ étant autre que le radical monovalent de l'hémoglobine;
- Z′ est l'unité saccharidique terminale réductrice du polysaccharide Oside-Z dans sa forme aldéhydique, soit naturelle, soit provenant de la dépolymérisation nitreuse, privée du groupe aldéhydique de Z;
- Oside est le reste du polysaccharide Oside-Z;
- n est un nombre entier de 2 à 30;

ou, le cas échéant un de ses sels pharmaceutiquement acceptables,

caractérisé en ce qu'on traite un polypeptide P représenté par la formule

$P°(NH_2)m$ (II)

dans laquelle P° est le radical m-valent du polypeptide P auquel sont liés ses m groupes fonctionnels $NH_2$ libres, lesdits m groupes fonctionnels $NH_2$ étant le groupe amino terminal et les groupes amino en position epsilon des (m-1) lysines contenues dans le polypeptide, avec un excès d'un polyoside représenté par la formule

Oside-Z (III)

Z étant l'unité saccharidique terminale réductrice dudit polysaccharide dans sa forme aldéhydique soit naturelle, soit provenant d'une dépolymérisation nitreuse et Oside étant le reste de la chaîne dudit polysaccharide et on soumet la poly-base de Schiff ainsi obtenue de formule

$(Oside\text{-}Z'\text{-}CH{=}N\text{-})_nP'$ (IV)

dans laquelle le radical monovalent Oside-Z'- représente le polysaccharide Oside-Z tel que défini ci-dessus, privé du groupe aldéhydique de Z, et P' et n sont tels que définis dans la revendication 1, à une réduction avec un cyanoborohydrure.

2. Procédé selon la revendication 1, caractérisé en ce que, comme polyoside Oside-Z de départ, on utilise un mélange de fragments d'héparine obtenus par dépolymérisation nitreuse ayant une masse moléculaire moyenne se situant entre 2000 et 8000 daltons.

3. Procédé selon l'une quelconque des revendications 1 et 2, caractérisé en ce que, comme polyoside Oside-Z de départ, on utilise un mélange de fragments d'héparine obtenus par dépolymérisation nitreuse ayant une masse moléculaire moyenne d'environ 4500 daltons.

4. Procédé selon l'une quelconque des revendications 1 à 3, caractérisé en ce que, comme cyanoborohydrure, on utilise le cyanoborohydrure de sodium.

5. Procédé selon l'un quelconque des revendications 1 à 4, caractérisé en ce que, comme cyanoborohydrure, on utilise le cyano-borohydrure de sodium, la réaction entre $P°(NH_2)m$ et Oside-Z étant conduite en présence dudit cyanoborohydrure de sodium de façon à obtenir directement le N-polyosyl-polypeptide final.

6. Procédé selon la revendication 1, caractérisé en ce que dans la formule (I) P' est le radical n-valent d'un polypeptide P choisi parmi les hormones peptidiques glycosylées ou non, les toxines glycosylées ou non, les enzymes et les inhibiteurs d'enzyme.

7. Procédé selon la revendication 1, caractérisé en ce que dans la formule (I) Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

8. Procédé selon l'une quelconque des revendications 1 à 7 caractérisé en ce que dans la formule (I) Z' a la structure

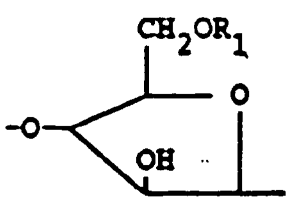

où $R_1$ est l'hydrogène ou un groupe $SO_3^-$.

9. Procédé selon l'une quelconque des revendications 1 à 8, caractérisé en ce que dans la formule (I)
   - P' est le radical n-valent d'un enzyme thrombolytique lié aux n groupes NH provenant de n groupes amino libres présents à l'origine dans la molécule dudit enzyme thrombolytique;
   - n est de 2 à 10.

10. Procédé selon la revendication 1, caractérisé en ce que dans la formule (I)
   - P' est le radical n-valent de l'urokinase lié aux n groupes NH provenant de n groupes amino libres

présents à l'origine dans la molécule de ladite urokinase;
- n est égal ou inférieur à 6, de préférence de 6,4 ou 3.

**11.** Procédé selon la revendication 1, caractérisé en ce que dans la la formule (I)
- P' est le radical n-valent de la streptokinase lié aux n groupes NH provenant de n groupes amino libres présents à l'origine dans la molécule de ladite streptokinase,
- n est de 2 à 4.

**12.** Procédé selon la revendication 1, caractérisé en ce que dans la la formule (I)
- P' est le radical n-valent du tPA lié aux n groupes NH provenant de n groupes amino libres présents à l'origine dans la molécule dudit tPA;
- n est de 3 à 10.

**13.** Procédé selon la revendication 1, caractérisé en ce qu'on prépare un N-polyosyl-polypeptide de formule

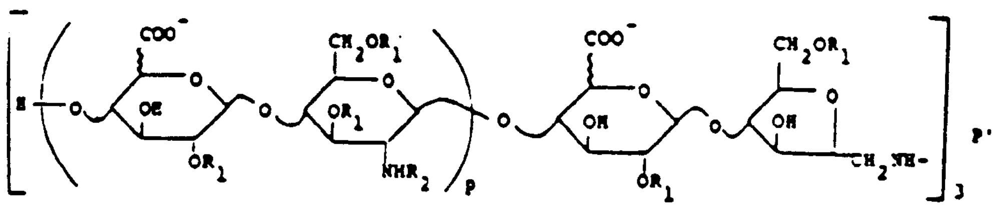

dans laquelle
- $R_1$ est l'hydrogène ou un groupe $SO_3^-$,
- $R_2$ est un groupe acétyle ou un groupe $SO_3^-$,
- P' est le radical trivalent de l'urokinase lié aux 3groupes NM provenant de 3 groupes amino libres présents à l'origine dans la molécule de l'urokinase, et
- p, de 4 à 24, représente le nombre d'unités disaccharidiques de fragments d'héparine provenant d'une dépolymérisation nitreuse dans un mélange ayant une masse moléculaire moyenne d'environ 4500 daltons et contenant le site de fixation à l'AT III.

**14.** Procédé selon la revendication 1, caractérisé en ce qu'on prépare un N-polyosyl-polypeptide de formule

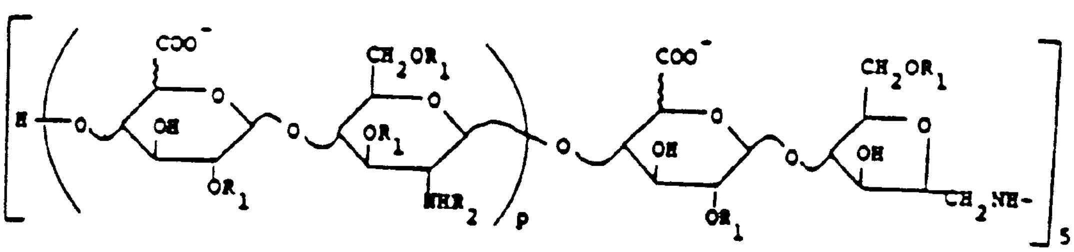

dans laquelle
- $R_1$ est l'hydrogène ou un groupe $SO_3^-$,
- $R_2$ est un groupe acétyle ou un groupe $SO_3^-$,
- P' est le radical pentavalent du tPA lié aux 5 groupes NH provenant de 5 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire d'environ 4500 daltons et contenant le site de fixation à l'AT III.

**15.** Procédé selon la revendication 1, caractérisé en ce qu'on prépare un N-polyosyl-polypeptide de formule

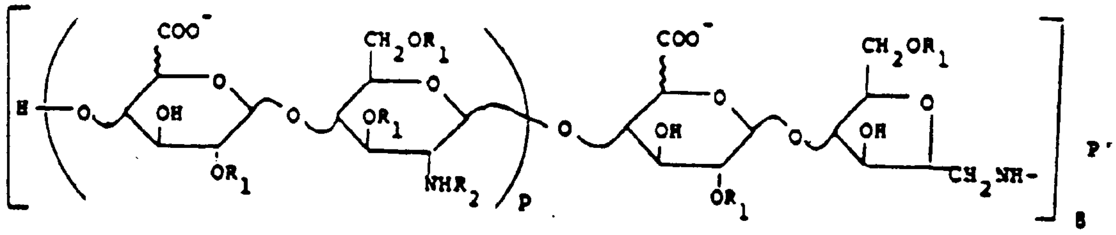

dans laquelle

- $R_1$ est l'hydrogène ou un groupe $SO_3^-$,
- $R_2$ est un groupe acétyle ou un groupe $SO_3^-$,
- P′ est le radical octovalent du tPA lié aux 8 groupes NH provenant de 8 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire d'environ 4500 daltons et contenant le site de fixation à l'AT III.

**16.** Procédé selon la revendication 1, caractérisé en ce qu'on prépare un N-polyosyl-polypeptide de formule

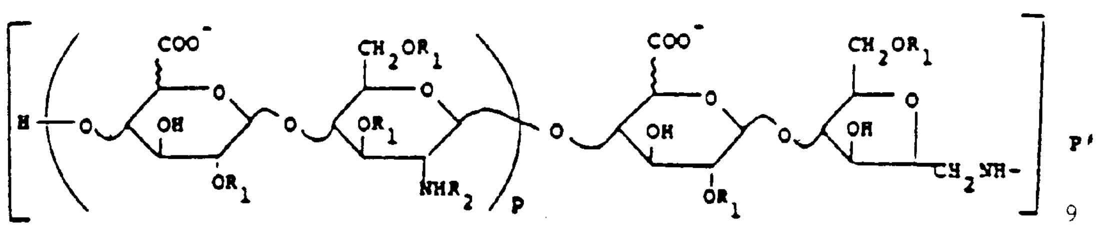

dans laquelle

- $R_1$ est l'hydrogène ou un groupe $SO_3^-$,
- $R_2$ est un groupe acétyle ou un groupe $SO_3^-$,
- P′ est le radical nonavalent du tPA lié aux 9 groupes NH provenant de 9 groupes amino libres présents à l'origine dans la molécule du tPA, et
- p, de 4 à 24, représente le nombre d'unités disaccharidiques de fragments d'héparine provenant d'une dépolymérisation nitreuse dans un mélange ayant une masse moléculaire d'environ 4500 daltons et contenant le site de fixation à l'AT III.

**17.** Réactif biologique constitué par un produit obtenu par le procédé selon l'une quelconque des revendications 1 à 16.

## Patentansprüche

### Patentansprüche für folgende Vertragsstaaten : AT, BE, CH, DE, FR, GB, GR, IT, LU, NL, SE, LI

**1.** N-Polyosyl-polypeptid der Formel

$$(Osid\text{-}Z'\text{-}CH_2\text{-}NH\text{-})_nP' \qquad (I),$$

worin

- P′ der n-wertige Rest eines Polypeptids p ist, der über die n NH-Gruppen aus n freien Aminogruppen gebunden ist, die ursprünglich im Molekül von P vorliegen, wobei P′ nicht der einwertige Hämoglobinrest ist;
- Z′ die terminale reduzierende Saccharideinheit des Polysaccharids Osid-Z in der Aldehydform natürlichen Ursprungs oder aus der Depolymerisation mit Salpetersäure ist und die Aldehydgruppe von Z nicht aufweist;
- Osid der Rest des Polysaccharids Osid-Z ist;

- n eine ganze Zahl von 2 bis 30 ist,
  oder nötigenfalls eines seiner pharmazeutisch akzeptablen Salze.

2. N-Polyosyl-peptid nach Anspruch 1, worin P′ der n-wertige Rest eines Polypeptids P ist, das ausgewählt wird unter glycosylierten oder unglycosylierten Peptidhormonen, glycosylierten oder unglycosylierten Toxinen, Enzymen und Enzyminhibitoren.

3. N-Polyosyl-polypeptid nach Anspruch 1 oder 2, gekennzeichnet durch die oben angegebene Formel (I), worin Osid den Rest eines Glycosaminglycans Osid-Z bedeutet, das ausgewählt wird unter Heparin, Heparinfragmenten, 4-S Chondroitinsulfat, 6-S Chondroitinsulfat, Heparansulfat und Dermatansulfat, das keine terminale reduzierende Einheit Z aufweist.

4. N-Polyosyl-polypeptid nach einem der Ansprüche 1 bis 3, gekennzeichnet durch die Formel (I), worin P′ und n das gleiche bedeuten wie in Anspruch 1;
   - Z′ die terminale reduzierende Saccharideinheit eines Glycosaminglycans auf der Basis von heparinischem Osid-Z in der Aldehydform aus der Depolymerisation mit Salpetersäure von Heparin ist, wobei das Osid-Z aus einem Gemisch von Heparinfragmenton mit einer mittleren Molekülmasse zwischen 2000 und 8000 Dalton besteht;
   - Osid der Rest des Polysaccharids Osid-Z ist.

5. N-Polyosyl-polypeptid nach einem der Ansprüche 1 bis 4, gekennzeichnet durch die Formel (I), worin P′ und n das gleiche bedeuten wie in Anspruch 1;
   - Z′ die terminale reduzierende Saccharideinheit eines Glycosaminglucans auf der Basis von heparinischem Osid-Z in der Aldehydform aus der Depolymerisation mit Salpetersäure von Heparin ist, wobei das Osid-Z aus einem Gemisch von Heparinfragmenten mit einer mittleren Molekülmasse von ungefähr 4500 Dalton besteht;
   - das Osid der Rest des Polysaccharids Osid-Z ist.

6. N-Polyosyl-polypeptid nach einem der Ansprüche 1 bis 5, gekennzeichnet durch die Formel (I), worin Z′ die folgende Struktur besitzt:

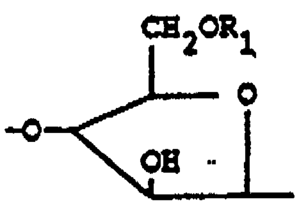

worin $R_1$ Wasserstoff oder eine $SO_3^-$-Gruppe ist.

7. N-Polyosyl-polypeptid nach einem dar Ansprüche 1 bis 6, gekennzeichnet durch die Formel (I), worin
   - P′ der n-wertige Rest eines thrombolytischen Enzyms ist, der über n NH-Gruppen gebunden ist, die aus n freien Aminogruppen stammen, die ursprünglich im Molekül des thrombolytischen Enzyms vorliegen;
   - n eine Zahl von 2 bis 10 ist.

8. N-Polyosyl-polypeptid nach Anspruch 7, gekennzeichnet durch die Formel (I), worin
   - P′ der n-wertige Rest von Urokinase ist, der über n NH-Gruppen gebunden ist, die aus n freien Aminogruppen stammen, die ursprünglich im Urokinasemolekül vorliegen;
   - n gleich oder kleiner 6, vorzugsweise 6, 4 oder 3 ist.

9. N-Polyosyl-polypeptid nach Anspruch 7, gekennzeichnet durch die Formel (I), worin
   - P′ der n-wertige Rest von Streptokinase ist, der über n NH-Gruppen gebunden ist, die aus n freien Aminogruppen stammen, die ursprünglich im Streptokinasemolekül vorliegen;
   - n eine Zahl von 2 bis 4 ist.

10. N-Polyosyl-polypeptid nach Anspruch 7, gekennzeichnet durch die Formel (I), worin

- P′ der n-wertige Rest von tPA ist, der über n NH-Gruppen gebunden ist, die aus n freien Aminogruppen stammen, die ursprünglich im tPA-Molekül vorliegen;
- n eine Zahl von 3 bis 10 ist.

**11.** N-Polyosyl-polypeptid der Formel

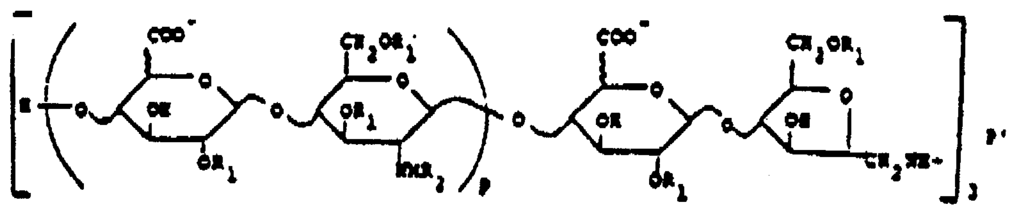

worin
- $R_1$ Wasserstoff oder eine $SO_3^-$-Gruppe ist;
- $R_2$ eine Acetylgruppe oder eine $SO_3^-$-Gruppe ist;
- P′ der dreiwertige Rest von Urokinase ist, der über drei NH-Gruppen gebunden ist, die aus drei freien Aminogruppen stammen, die ursprünglich im Urokinasemolekül vorliegen und worin
- p eine Zahl von 4 bis 24 ist, und die Anzahl der Disaccharideinheiten von Heparinfragmenten aus einer Depolymerisation mit Salpetersäure in einem Gemisch mit einer mittleren Molekülmasse von ungefähr 4500 Dalton darstellt und die die Bindungsstelle an das AT III enthalten.

**12.** N-Polyosyl-polypeptid der Formel

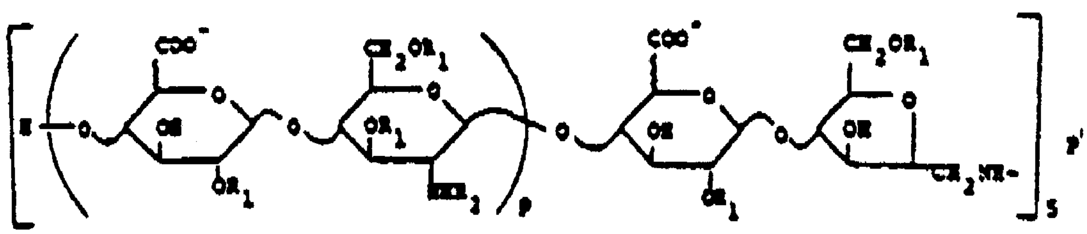

worin
- $R_1$ Wasserstoff oder eine $SO_3^-$-Gruppe ist;
- $R_2$ eine Acetylgruppe oder eine $SO_3^-$-Gruppe ist;
- P′ der fünfwertige Rest von tPA ist, der über fünf NH-Gruppen gebunden ist, die aus fünf freien Aminogruppen stammen, die ursprünglich im tPA-Molekül vorliegen und
- p, das eine Zahl von 4 bis 24 ist, die Anzahl der Disaccharideinheiten von Heparinfragmenten aus einer Depolymerisation mit Salpetersäure in einem Gemisch mit einer mittleren Molekülmasse von ungefähr 4500 Dalton darstellt und die die Bindungsstelle an das AT III enthalten.

**13.** N-Polyosyl-polypeptid der Formel

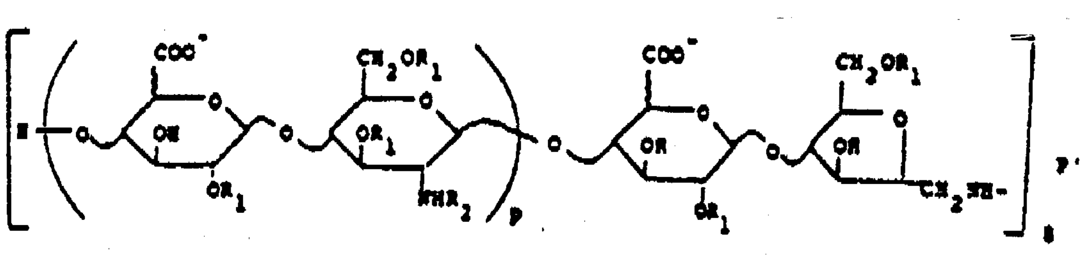

worin

- $R_1$ Wasserstoff oder eine $SO_3^-$-Gruppe ist;
- $R_2$ eine Acetylgruppe oder eine $SO_3^-$-Gruppe ist;
- P′ der achtwertige Rest von tPA ist, der über acht NH-Gruppen gebunden ist, die aus acht freien Aminogruppen stammen, die ursprünglich im tPA-Molekül vorliegen und
- p, das eine Zahl von 4 bis 24 ist, die Anzahl der Disaccharideinheiten von Heparinfragmenten aus einer Depolymerisation mit Salpetersäure in einem Gemisch mit einer Molekülmasse von ungefähr 4500 Dalton darstellt und die die Bindungsstelle an das AT III enthält.

**14.** N-Polyosyl-polypeptid der Formel

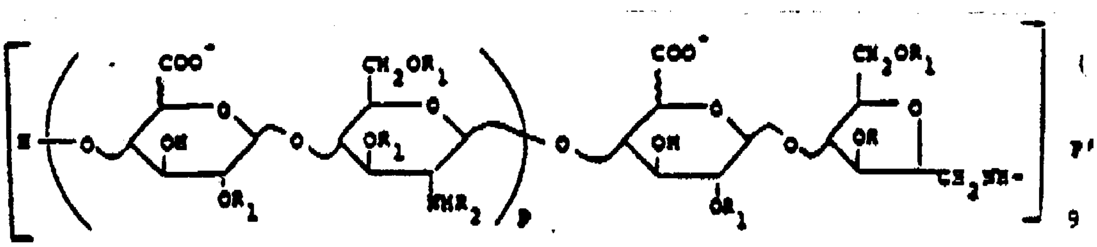

worin
- $R_1$ Wasserstoff oder eine $SO_3^-$-Gruppe ist;
- $R_2$ eine Acetylgruppe oder eine $SO_3^-$-Gruppe ist;
- P′ der neunwertige Rest von tPA ist, der über neun NH-Gruppen gebunden ist, die aus neun freien Aminogruppen stammen, die ursprünglich im tPA-Molekül vorliegen und
- p, das eine Zahl von 4 bis 24 ist, die Anzahl der Disaccharideinheiten von Heparinfragmenten aus einer Depolymerisation mit Salpetersäure in einem Gemisch mit einer Molekülmasse von ungefähr 4500 Dalton darstellt, und die die Bindungsstelle an das AT III enthalten.

**15.** Verfahren zur Herstellung eines N-Polyosyl-polypeptids nach Anspruch 1, dadurch gekennzeichnet, daß ein Polypeptid P der Formel (II)

$$P(NH_2)m \qquad (II)$$

worin P° der m-wertige Rest des Polypeptids P ist, an den m funktionelle frei $NH_2$-Gruppen gebunden sind, wobei die m funktionellen $NH_2$-Gruppen die terminale Aminogruppe und die Aminogruppe in der ε-Position der (m-1) im Polypeptid enthaltenden Lysingruppen sind, mit einem Überschuß eines Polyosids der allgemeinen Formel (III)

$$\text{Osid-Z} \qquad (III)$$

behandelt wird,
wobei Z die terminale reduzierende Saccharideinheit des Polysaccharids in der Aldehydform entweder natürlichen Ursprungs oder aus der Depolymerisation mit Salpetersäure ist und wobei Osid der Rest der Polysaccharidkette ist, und wobei die so erhaltene Schiffpolybase der Formel

$$(\text{Osid-Z}'\text{-CH=N-})_nP' \qquad (IV),$$

worin der einwertige Rest Osid-Z′ das Polysaccharid Osid-Z wie oben definiert ohne die Aldehydgruppe von Z darstellt, und P′ und n das gleiche bedeuten wie in Anspruch 1,
mit Cyanoborhydrid reduziert wird.

**16.** Verfahren nach Anspruch 15, dadurch gekennzeichnet, daß als Ausgangs-Polyosid Osid-Z ein Gemisch von Heparinfragmenten, aus der Depolymerisation mit Salpetersäure mit einer mittleren Molekülmasse zwischen 2000 und 8000 Dalton verwendet wird.

**17.** Verfahren nach einem der Ansprüche 15 und 16, dadurch gekennzeichnet, daß als Ausgangspolyosid Osid-Z ein Gemisch von Heparinfragmenten aus der Depolymerisation mit Salpetersäure verwendet wird, die eine mittlere Molekülmasse von ungefähr 4500 Dalton besitzen.

**18.** Verfahren nach einem der Ansprüche 15 bis 17, dadurch gekennzeichnet, daß als Cyanoborhydrid Natriumcyanoborhydrid verwendet wird.

**19.** Verfahren nach einem der Ansprüche 15 bis 18, dadurch gekennzeichnet, daß als Cyanoborhydrid Natriumcyanoborhydrid verwendet wird, wobei die Reaktion zwischen $P°(NH_2)_m$ und Osid-Z in Gegenwart

des Natriumcyanoborhydrids so durchgeführt wird, daß das gewünschte N-Polyosyl -polypeptid direkt erhalten wird.

**20.** Schiffpolybase der Formel

$$(\text{Osid-Z}'\text{-CH=N-})_n\text{P}' \qquad (\text{IV})$$

worin

- P′ der n-wertige Rest eines Polypeptids P ist, der über die n NH-Gruppen aus n freien Aminogruppen gebunden ist, die ursprünglich im Molekül von P vorliegen, wobei P′ nicht der einwertige Hämoglobinrest ist;
- Z′ die terminale reduzierende Saccharideinheit des Polysaccharids Osid-Z in dar Aldehydform ist, die entweder natürlich ist oder aus der Depolymerisation mit Salpetersäure stammt und die Aldehydgruppe von Z nicht enthält;
- Osid der Rest des Polysaccharids Osid-Z ist;
- n eine ganze Zahl zwischen 2 und 30 ist.

**21.** Schiffpolybase nach Anspruch 20, dadurch gekennzeichnet, daß P′ der n-wertige Rest eines Polypeptids P ist, das ausgewählt wird unter glycosylierten oder unglycosylierten Peptidhormonen, glycosylierten oder unglycosylierten Toxinen, Enzymen und Enzyminhibitoren.

**22.** Schiffpolybase nach den Ansprüchen 20 oder 21, gekennzeichnet durch die oben angegebene Formel (IV), worin Osid den Rest eines Glycosaminglucans Osid-Z darstellt, das ausgewählt wird unter Heparin, Heparinfragmenten, 4-S-Chondroitinsulfat, 6-S-Chondroitinsulfat, Heparansulfat und Dermatansulfat und keine terminale reduzierende Einheit Z aufweist.

**23.** Schiffpolybase nach Anspruch 20, gekennzeichnet durch die Formel (IV), worin

- Z′ die terminale reduzierende Polysaccharideinheit eines Glycosaminglucans auf der Basis von Heparin-Osid-Z in der Aldehydform aus der Depolymerisation mit Salpetersäure von Heparin ist, wobei das Osid-Z aus einem Gemisch von Heparinfragmenten mit einer mittleren Molekülmasse zwischen 2000 und 8000 Dalton besteht;
- Osid der Rest des Polysaccharids Osid-Z ist.

**24.** Schiffpolybase nach Anspruch 20, gekennzeichnet durch die Formel (IV), worin P′ und n das gleiche bedeuten wie in Anspruch 20;

- Z′ eine terminale reduzierende Saccharideinheit eines Glycosaminglucans auf der Basis von heparinischem Osid-Z in der Aldehydform aus der Depolymerisation mit Salpetersäure von Heparin ist, wobei das Osid-Z aus einem Gemisch von Heparinfragmenten einer mittleren Molekülmasse von ungefähr 4500 Dalton besteht;
- Osid der Rest des Polysaccharids Osid-Z ist.

**25.** Schiffpolybase nach einem der Ansprüche 20 bis 24, gekennzeichnet durch die Formel (IV), worin Z′ die folgende Struktur besitzt

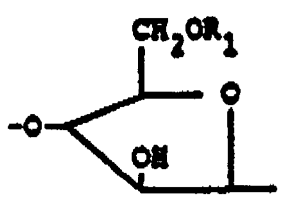

worin $R_1$ Wasserstoff oder eine $SO_3^-$-Gruppe bedeutet.

**26.** Schiffpolybase nach einem der Ansprüche 20 bis 25, gekennzeichnet durch die Formel (IV), worin

- P′ der n-wertige Rest eines thrombolytischen Enzyms ist, der über n NH-Gruppen gebunden ist, die aus den n freien Aminogruppen stammen, die ursprünglich im thrombolytischen Enzym vorliegen;
- n eine Zahl von 2 bis 10 ist.

**27.** Schiffpolybase nach Anspruch 26, gekennzeichnet durch die Formel (IV), worin

- P′ der n-wertige Rest von Urokinase ist, der über n NH-Gruppen gebunden ist, die aus n freien Aminogruppen stammen, die ursprünglich im Urokinasemolekül vorliegen;
- n gleich oder kleiner 6 ist, vorzugsweise 6, 4 oder 3.

**28.** Schiffpolybase nach Anspruch 26, gekennzeichnet durch Formel (IV), worin
- P′ der n-wertige Rest von Streptokinase ist, der über n NH-Gruppen gebunden ist, die aus n freien Aminogruppen stammen, die ursprünglich im Streptokinasemolekül vorliegen;
- n eine Zahl von 2 bis 4 ist.

**29.** Schiffpolybase nach Anspruch 26, gekennzeichnet durch Formel (IV), worin
- P′ der n-wertige Rest von tPA ist, der über n NH-Gruppen gebunden ist, die aus n freien Aminogruppen stammen, die ursprünglich im tPA-Molekül vorliegen;
- n eine Zahl von 3 bis 10 ist.

**30.** Schiffpolybase der Formel

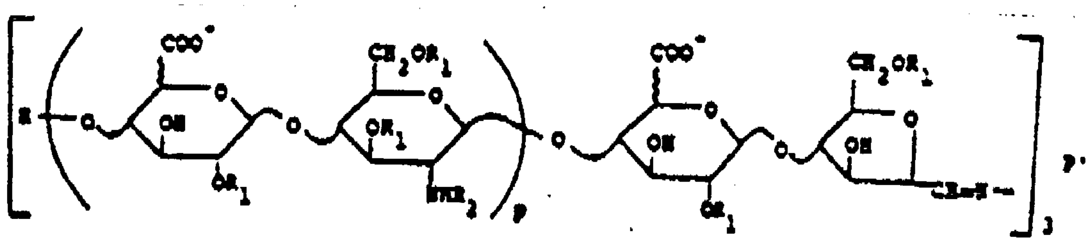

worin
- $R_1$ Wasserstoff oder eine $SO_3$-Gruppe ist;
- $R_2$ eine Acetylgruppe oder eine $SO_3$-Gruppe ist;
- P′ der dreiwertige Rest von Urokinase ist, der über drei NH-Gruppen gebunden ist, die aus drei freien Aminogruppen stammen, die ursprünglich im Urokinasemolekül vorliegen und worin
- p, das eine Zahl von 4 bis 24 ist, die Anzahl der Disaccharideinheiten der Heparinfragmente aus einer Depolymerisation mit Salpetersäure in einem Gemisch mit einer mittleren Molekülmasse von ungefähr 4500 Dalton darstellt, die die Bindungsstelle an das AT III enthalten.

**31.** Schiffpolybase der Formel

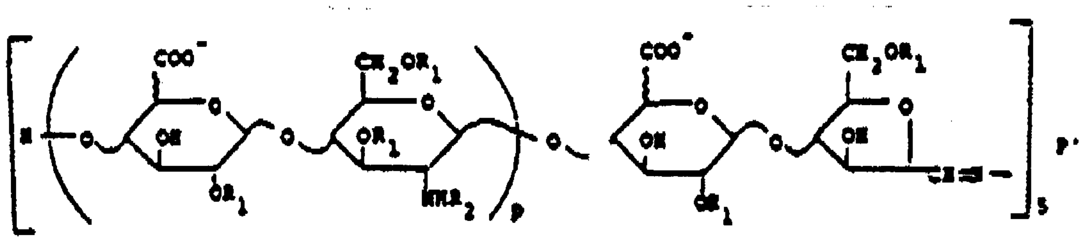

worin
- $R_1$ Wasserstoff oder eine $SO_3^-$-Gruppe ist;
- $R_2$ eine Acetylgruppe oder eine $SO_3^-$-Gruppe ist;
- P′ der fünfwertige Rest von tPA ist, der über fünf NH-Gruppen gebunden ist, die aus fünf freien Aminogruppen stammen, die ursprünglich im tPA-Molekül vorliegen und
- p, das eine Zahl von 4 bis 24 ist, die Anzahl der Disaccharideinheiten der Heparinfragmente aus einer Depolymerisation mit Salpetersäure in einem Gemisch mit einer mittleren Molekülmasse von ungefähr 4500 Dalton darstellt, die die Bindungsstelle an das AT III enthalten.

**32.** Schiffpolybase der Formel

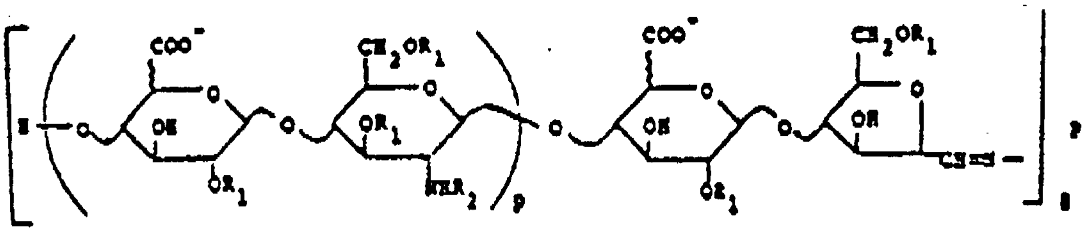

worin
- $R_1$ Wasserstoff oder eine $SO_3^-$-Gruppe ist;
- $R_2$ eine Acetylgruppe oder eine $SO_3^-$-Gruppe ist;
- P′ der achtwertige Rest des tPA ist, der über acht NH-Gruppen gebunden ist, die aus acht freien Aminogruppen stammen, die ursprünglich im tPA-Molekül vorliegen und
- p, das eine Zahl von 4 bis 24 ist, die Anzahl der Disaccharideinheiten der Heparinfragmente aus einer Depolymerisation mit Salpetersäure in einem Gemisch mit einer mittleren Molekülmasse von ungefähr 4500 Dalton darstellt, die die Bindungsstelle an das AT III enthalten.

**33.** Schiffpolybase der Formel

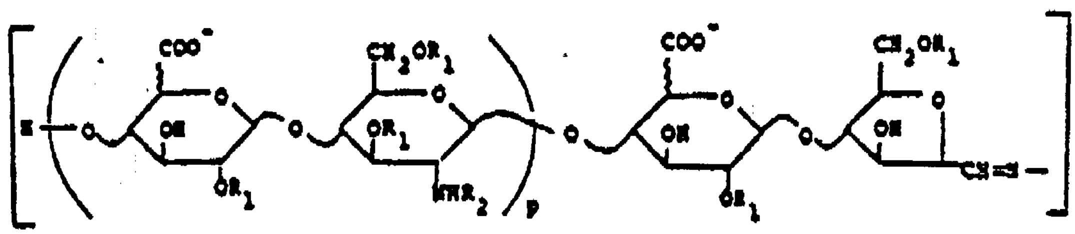

worin
- $R_1$ Wasserstoff oder eine $SO_3^-$-Gruppe ist;
- $R_2$ eine Acetylgruppe oder eine $SO_3^-$-Gruppe ist;
- P′ der einwertige Rest von tPA ist, der über neun NH-Gruppen gebunden ist, die aus neun freien Aminogruppen stammen, die ursprünglich im tPA-Molekül vorliegen und
- p, das eine Zahl zwischen 4 bis 24 ist, die Anzahl der Disaccharideinheiten der Heparinfragmente aus einer Depolymerisation mit Salpetersäure in einem Gemisch mit einer mittleren Molekülmasse von ungefähr 4500 Dalton darstellt, die die Bindungsstelle an das AT III enthalten.

**34.** Pharmazeutische Zusammensetzung, dadurch gekennzeichnet, daß sie als Wirkstoff eine wirksame Menge von mindestens einem N-Polyosid-polypeptid nach einem dar Ansprüche 1 bis 14 zusammen mit einem pharmazeutischen Träger enthält.

**35.** Biologisches Reagens, das aus einem Produkt nach einem der Ansprüche 1 bis 14 besteht.

**Patentansprüche fur folgenden Vertragsstaat : ES**

**1.** Verfahren zur Herstellung eines N-Polyosyl-polypeptids der Formel

$$(\text{Osid-Z}'\text{-CH}_2\text{-NH-})_n\text{P}' \quad \text{(I)},$$

worin
- P′ der n-wertige Rest eines Polypeptids P ist, der über die n NH-Gruppen aus n freien Aminogruppen gebunden ist, die ursprünglich im Molekül von P vorliegen, wobei P′ nicht der einwertige Hämoglobinrest ist;
- Z′ die terminale reduzierende Saccharideinheit des Polysaccharids Osid-Z in der Aldehydform natürlichen Ursprungs oder aus der Depolymerisation mit Salpetersäure ist und die Aldehydgruppe von Z nicht aufweist;
- Oxid der Rest des Polysaccharids Osid-Z ist;
- n eine ganze Zahl von 2 bis 30 ist,

oder nötigenfalls eines seiner pharmazeutisch akzeptablen Salze,

dadurch gekennzeichnet, daß ein polypeptid P der Formel (II)

$$P°(NH_2)m \qquad (II)$$

worin P° der m-wertige Rest des Polypeptids P ist, an den m funktionelle frei $NH_2$-Gruppen gebunden sind, wobei die m funktionellen $NH_2$-Gruppen die terminale Aminogruppe und die Aminogruppe in der ε-Position der (m-1) im Polypeptid enthaltenden Lysingruppen sind, mit einem Überschuß eines Polyosids der allgemeinen Formel (III)

$$\text{Osid-Z} \qquad (III)$$

behandelt wird,
wobei Z die terminale reduzierende Saccharideinheit des Polysaccharids in der Aldehydform entweder natürlichen Ursprungs oder aus der Depolymerisation mit Salpetersäure ist und wobei Osid der Rest der Polysaccharidkette ist und wobei die so erhaltene Schiffpolybase der Formel

$$(\text{Osid-Z}'\text{-CH=N-})_nP' \qquad (IV),$$

worin der einwertige Rest Osid-Z′ das Polysaccharid Osid-Z wie oben definiert ohne die Aldehydgruppe von Z darstellt, und P′ und n das gleiche bedeuten wie oben, mit Cyanoborhydrid reduziert wird.

2. Verfahren nach Anspruch 1, dadurch gekennzeichnet, daß als Ausgangs-Polyosid Osid-Z ein Gemisch von Heparinfragmenten aus der Depolymerisation mit Salpetersäure mit einer mittleren Molekülmasse zwischen 2000 und 8000 Dalton verwendet wird.

3. Verfahren nach einem der Ansprüche 1 und 2, dadurch gekennzeichnet, daß als Ausgangspolyosid Osid-Z ein Gemisch von Heparinfragmenten aus der Depolymerisation mit Salpetersäure verwendet wird, die eine mittlere Molekülmasse von ungefähr 4500 Dalton besitzen.

4. Verfahren nach einem der Ansprüche 1 bis 3, dadurch gekennzeichnet, daß als Cyanoborhydrid Matriumcyanoborhydrid verwendet wird.

5. Verfahren nach einem der Ansprüche 1 bis 4, dadurch gekennzeichnet, daß als Cyanoborhydrid Natriumcyanoborhydrid verwendet wird, wobei die Reaktion zwischen $P°(NH_2)_m$ und Osid-Z in Gegenwart des Natriumcyanoborhydrids so durchgeführt wird, daß das gewünschte N-Polyosyl-polypeptid direkt erhalten wird.

6. Verfahren nach Anspruch 1, dadurch gekennzeichnet, daß in Formel (I) P′ der n-wertige Rest eines Polypeptids P ist, das ausgewählt wird unter glycosylierten oder unglycosylierten Peptidhormonen, glycosylierten oder unglycosylierten Toxinen, Enzymen und Enzyminhibitoren.

7. Verfahren nach Anspruch 1, dadurch gekennzeichnet, daß in Formel (I), Osid den Rest eines Glycosaminglycans Osid-Z bedeutet, das ausgewählt wird unter Heparin, Heparinfragmenten, 4-S Chondroitinsulfat, 6-S Chondroitinsulfat, Heparansulfat und Dermatansulfat, das keine terminale reduzierende Einheit Z aufweist.

8. Verfahren nach einem der Ansprüche 1 bis 7, dadurch gekennzeichnet, daß in Formel (I) Z′ die folgende Struktur besitzt:

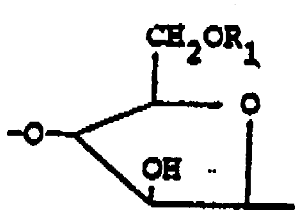

worin $R_1$ Wasserstoff oder eine $SO_3^-$-Gruppe ist.

9. Verfahren nach einem der Ansprüche 1 bis 8, dadurch gekennzeichnet, daß in Formel (I)
- P′ der n-wertige Rest eines thrombolytischen Enzyms ist, der über n NH-Gruppen gebunden ist, die aus n freien Aminogruppen stammen, die ursprünglich im Molekül des thrombolytischen Enzyms vorliegen;
- n eine Zahl von 2 bis 10 ist.

10. Verfahren nach Anspruch 1, dadurch gekennzeichnet, daß in Formel (I)
- P′ der n-wertige Rest von Urokinase ist, der über n NH-Gruppen gebunden ist, die aus n freien Aminogruppen stammen, die ursprünglich im Urokinasemolekül vorliegen;
- n gleich oder kleiner 6, vorzugsweise 6, 4 oder 3 ist.

11. Verfahren nach Anspruch 1, dadurch gekennzeichnet, daß in Formel (I)
- P′ der n-wertige Rest von Streptokinase ist, der über n NH-Gruppen gebunden ist, die aus n freien Aminogruppen stammen, die ursprünglich im Streptokinasemolekül vorliegen;
- n eine Zahl von 2 bis 4 ist.

12. Verfahren nach Anspruch 1, dadurch gekennzeichnet, daß in Formel (I)
- P′ der n-wertige Rest von tPA ist, der über n NH-Gruppen gebunden ist, die aus n freien Aminogruppen stammen, die ursgrünglich im tPA-Molekül vorliegen;
- n eine Zahl von 3 bis 10 ist.

13. Verfahren nach Anspruch 1, dadurch gekennzeichnet, daß man ein N-Polyosyl-polypeptid der Formel

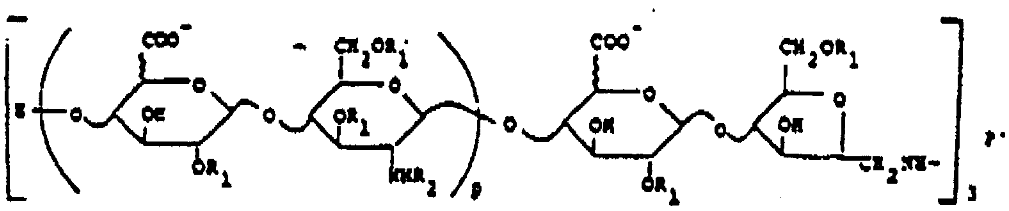

herstellt,
worin
- $R_1$ Wasserstoff oder eine $SO_3^-$-Gruppe ist;
- $R_2$ eine Acetylgruppe oder eine $SO_3^-$-Gruppe ist;
- P′ der dreiwertige Rest von Urokinase ist, der über drei NH-Gruppen gebunden ist, die aus drei freien Aminogruppen stammen, die ursprünglich im Urokinasemolekül vorliegen und worin
- p eine Zahl von 4 bis 24 ist, und die Anzahl der Disaccharideinheiten der Heparinfragmente aus einer Depolymerisation mit Salpetersäure in einem Gemisch mit einer mittleren Molekülmasse von ungefähr 4500 Dalton darstellt und die die Bindungsstelle an das AT III enthalten.

14. Verfahren nach Anspruch 1, dadurch gekennzeichnet, daß man ein N-Polyosyl-polypeptid der Formel

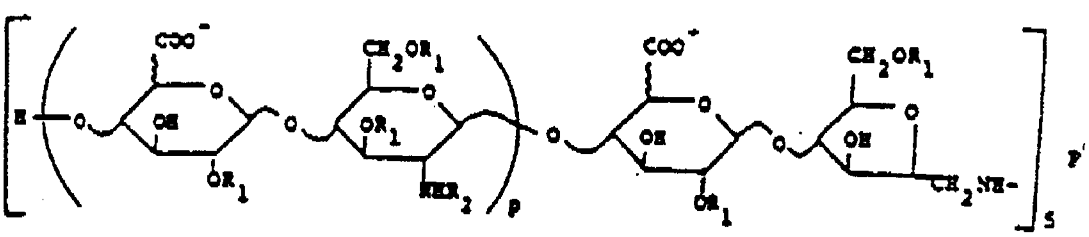

herstellt,
worin
- $R_1$ Wasserstoff oder eine $SO_3^-$-Gruppe ist;
- $R_2$ eine Acetylgruppe oder eine $SO_3^-$-Gruppe ist;
- P′ der fünfwertige Rest des tPA ist, der über fünf NH-Gruppen gebunden ist, die aus fünf freien Aminogruppen stammen, die ursprünglich im tPA-Molekül vorliegen und
- p, das eine Zahl zwischen 4 und 24 ist, die Anzahl der Disaccharideinheiten der Heparinfragmente aus einer Depolymerisation mit Salpetersäure in einem Gemisch mit einer mittleren Molekülmasse von ungefähr 4500 Dalton darstellt und die die Bindungsstelle an das AT III enthalten.

15. Verfahren nach Anspruch 1, dadurch gekennzeichnet, daß man ein N-Polyosyl-polypeptid der Formel

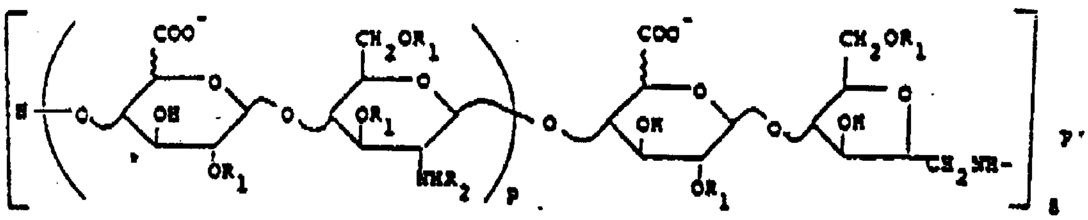

herstellt,
worin

- $R_1$ Wasserstoff oder eine $SO_3^-$-Gruppe ist;
- $R_2$ eine Acetylgruppe oder eine $SO_3^-$-Gruppe ist;
- P′ der achtwertige Rest des tpA ist, der an acht NH-Gruppen gebunden ist, die aus acht freien Aminogruppen stammen, die ursprünglich im tpA-Molekül vorliegen und
- p, das eine Zahl von 4 bis 24 ist, die Anzahl der Disaccharideinheiten von Heparinfragmenten aus einer Depolymerisation mit Salpetersäure in einem Gemisch mit einer Molekülmasse von ungefähr 4500 Dalton darstellt und die die Bindungsstelle an das AT III enthalten.

**16.** Verfahren nach Anspruch 1, dadurch gekennzeichnet, daß man ein N-Polyosyl-polypeptid der Formel

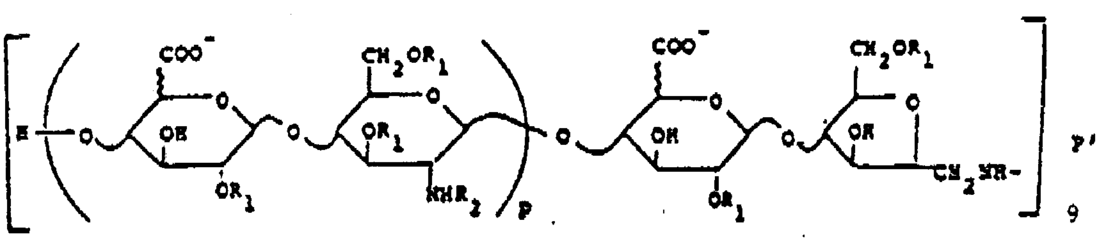

worin

- $R_1$ Wasserstoff oder eine $SO_3^-$-Gruppe ist;
- $R_2$ eine Acetylgruppe oder eine $SO_3^-$-Gruppe ist;
- P′ der neunwertige Rest des tPA ist, der an neun NH-Gruppen gebunden ist, die aus neun freien Aminogruppen stammen, die ursprünglich im tPA-Molekül vorliegen und
- p, das eine Zahl von 4 bis 24 ist, die Anzahl der Disaccharideinheiten von Heparinfragmenten aus einer Depolymerisation mit Salpetersäure in einem Gemisch mit einer Molekülmasse von ungefähr 4500 Dalton darstellt, und die die Bindungsstelle an das AT III enthalten,

herstellt.

**17.** Biologisches Reagens, das aus einem nach einem der Ansprüche 1 bis 6 erhaltenen Produkt besteht.

## Claims

**Claims for the following Contracting States : AT, BE, CH, DE, FR, GB, GR, IT, LU, NL, LI, SE**

**1.** N-polyosyl-polypeptide of formula

$$(Oside\text{-}Z'\text{-}CH_2\text{-}NH\text{-})_nP' \qquad (I)$$

wherein

- P′ is the n-valent radical of a polypeptide P linked to n NH groups coming from n free amino groups originally present in the P molecule, P′ being other than the monovalent radical of haemoglobin;
- Z′ is the terminal reducing saccharide unit of the polysaccharide Oside-Z in its aldehyde form, either natural or produced by nitrous depolymerisation, deprived of the aldehyde group of Z;
- Oside is the residue of the polysaccharide Oside-Z;
- n is an integer from 2 to 30;

or, optionally, one of the pharmaceutically acceptable salts thereof.

2. N-polyosyl-peptide according to claim 1 wherein P′ is the n-valent radical of a polypeptide P selected from the glycosylated or non-glycosylated peptide hormones, glycosylated or non-glycosylated toxins, enzymes and enzyme inhibitors.

3. N-polyosyl-polypeptide according to claim 1 or 2, characterised by formula (I) above, wherein Oside represents the radical of a glycosaminoglycane Oside-Z selected from heparin, heparin fragments, chondroitin sulphate 4-S, chondroitin sulphate 6-S, heparan sulphate and dermatan sulphate; deprived of the terminal reducing unit Z.

4. N-polyosyl-polypeptide according to any of claims 1 to 3, characterised by formula (I) wherein P′ and n are as defined in claim 1;
   - Z′ is the terminal reducing saccharide unit of a glycosaminoglycane of the heparin Oside-Z type in its aldehyde form produced by the nitrous depolymerisation of heparin, the said Oside-Z consisting of a mixture of heparin fragments having a mean molecular mass between 2000 and 8000 daltons;
   - Oside is the residue of the polysaccharide Oside-Z.

5. N-polyosyl-polypeptide according to any one of claims 1 to 4, characterised by formula (I) wherein P′ and n are as defined in claim 1;
   - Z′ is the terminal reducing saccharide unit of a glycosaminoglycane of the heparin Oside-Z type in its aldehyde form obtained by nitrous depolymerisation of heparin, the Oside-Z consisting of a mixture of heparin fragments having a mean molecular mass of about 4500 daltons;
   - Oside is the residue of the polysaccharide Oside-Z.

6. N-polyosyl-polypeptide according to any of claims 1 to 5, characterised by formula (I) wherein Z′ has the structure

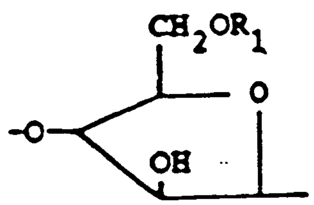

wherein $R_1$ is hydrogen or an $SO_3^-$ group.

7. N-polyosyl-polypeptide according to any one of claims 1 to 6, characterised by formula (I) wherein
   - P′ is the n-valent radical of a thrombolytic enzyme linked to n NH groups obtained from n free amino groups originally present in the molecule of said thrombolytic enzyme;
   - n is from 2 to 10.

8. N-polyosyl-polypeptide according to claim 7, characterised by formula (I) wherein
   - P′ is the n-valent radical of urokinase linked to n NH groups obtained from n free amino groups originally present in the molecule of said urokinase;
   - n is less than or equal to 6, preferably 6, 4 or 3.

9. N-polyosyl-polypeptide according to claim 7, characterised by formula (I) wherein
   - P′ is the n-valent radical of streptokinase linked to the n NH groups obtained from n free amino groups originally present in the molecule of said streptokinase,
   - n is from 2 to 4.

10. N-polyosyl-polypeptide according to claim 7, characterised by formula (I) wherein
   - P′ is the n-valent radical of tPA linked to the n NH groups obtained from n free amino groups originally present in the molecule of said tPA;
   - n is from 3 to 10.

11. N-polyosyl-polypeptide of formula

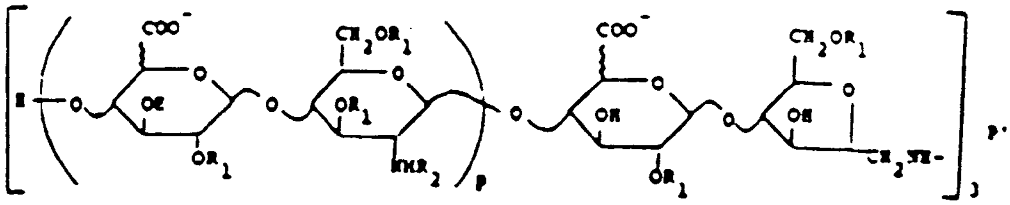

wherein
- $R_1$ is hydrogen or an $SO_3^-$ group,
- $R_2$ is an acetyl group or an $SO_3^-$ group,
- P′ is the trivalent radical of urokinase linked to the 3 NH groups obtained from 3 free amino groups originally present in the molecule of urokinase, and
- p, denoting from 4 to 24, represents the number of disaccharide units of heparin fragments obtained by nitrous depolymerisation in a mixture having a mean molecular mass of about 4500 daltons and containing the site for fixing to AT III.

**12.** N-polyosyl-polypeptide of formula

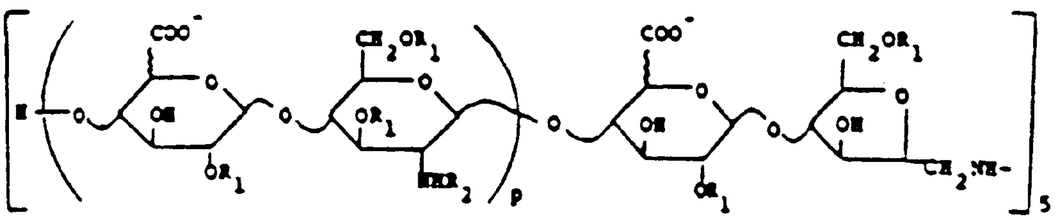

wherein
- $R_1$ is hydrogen or an $SO_3^-$ group,
- $R_2$ is an acetyl group or an $SO_3^-$ group,
- P′ is the pentavalent radical of tPA linked to the 5 NH groups obtained from 5 free amino groups originally present in the molecule of tPA, and
- p, denoting from 4 to 24, represents the number of disaccharide units of heparin fragments obtained from nitrous depolymerisation in a mixture having a molecular mass of about 4500 daltons and containing the site for fixing to AT III.

**13.** N-polyosyl-polypeptide of formula

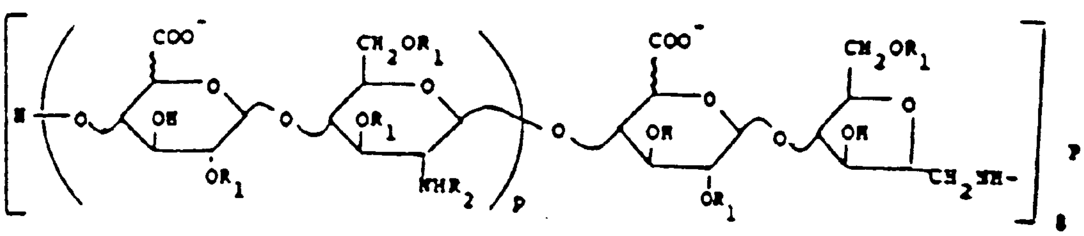

wherein
- $R_1$ is hydrogen or an $SO_3^-$ group,
- $R_2$ is an acetyl group or an $SO_3^-$ group,
- P′ is the octovalent radical of tPA linked to the 8 NH groups obtained from 8 free amino groups originally present in the molecule of tPA, and
- p, denoting from 4 to 24, represents the number of disaccharide units of heparin fragments obtained

by nitrous depolymerisation in a mixture having a molecular mass of about 4500 daltons and containing the site for fixing to AT III.

**14.** N-polyosyl-polypeptide of formula

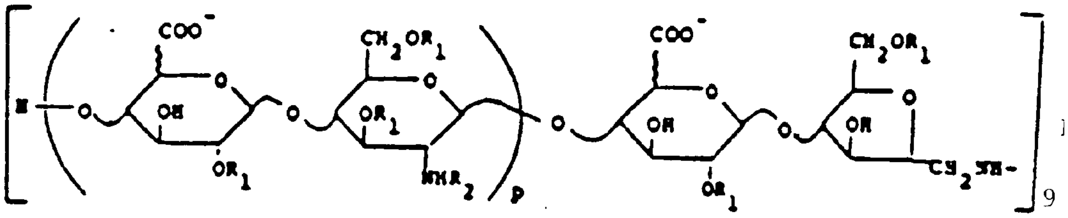

wherein

- $R_1$ is hydrogen or an $SO_3^-$ group,
- $R_2$ is an acetyl group or an $SO_3^-$ group,
- P′ is the nonavalent radical of tPA linked to the 9 NH groups obtained from 9 free amino groups originally present in the molecule of tPA, and
- p, denoting from 4 to 24, represents the number of disaccharide units of heparin fragments obtained by nitrous depolymerisation in a mixture having a molecular mass of about 4500 daltons and containing the site for fixing to AT III.

**15.** Process for preparing an N-polyosyl-polypeptide according to claim 1, characterised in that a polypeptide P represented by the formula

$$P^\circ(NH_2)_m \qquad (II)$$

wherein P° is the m-valent radical of the polypeptide P to which its m functional free $NH_2$ groups are linked, said m functional $NH_2$ groups being the terminal amino group and the amino groups in the epsilon position of the (m-1) lysines contained in the polypeptide, is treated with an excess of a polyoside represented by the formula

$$\text{Oside-Z} \qquad (III)$$

wherein Z is the terminal reducing saccharide unit of said polysaccharide in its aldehyde form which may either be natural or produced by nitrous depolymerisation and Oside is the residue of the chain of said polysaccharide, and the Schiff's polybase obtained of formula

$$(\text{Oside-Z}'\text{-CH=N-})_nP' \qquad (IV)$$

wherein the monovalent radical Oside-Z′- represents the polysaccharide Oside-Z as defined above, deprived of the aldehyde group of Z, and P′ and n are as defined in claim 1, is subjected to reduction with a cyanoborohydride.

**16.** Process according to claim 15, characterised in that the starting polyoside Oside-Z used consists of a mixture of heparin fragments obtained by nitrous depolymerisation having a mean molecular mass of between 2000 and 8000 daltons.

**17.** Process according to any one of claims 15 and 16, characterised in that the starting polyoside Oside-Z used consists of a mixture of heparin fragments obtained by nitrous depolymerisation having a mean molecular mass of about 4500 daltons.

**18.** Process according to any one of claims 15 to 17, characterised in that the cyanoborohydride used is sodium cyanoborohydride.

**19.** Process according to any one of claims 15 to 18, characterised in that the cyanoborohydride used is sodium cyanoborohydride, the reaction between $P^\circ(NH_2)_m$ and Oside-Z being performed in the presence of said sodium cyanoborohydride so that the final N-polyosylpolypeptide is obtained directly.

**20.** Schiff's polybase of formula

$$(\text{Oside-Z}'\text{-CH=N-})_nP' \qquad (IV)$$

wherein

- P′ is the n-valent radical of a polypeptide P linked to n NH groups coming from n free amino groups

originally present in the P molecule, P′ being other than the monovalent radical of haemoglobin,
- Z′ is the terminal reducing saccharide unit of the polysaccharide Oside-Z in its aldehyde form, either natural or obtained by nitrous depolymerisation, deprived of the aldehyde group of Z;
- Oside is the residue of the polysaccharide Oside-Z;
- n is an integer from 2 to 30.

**21.** Schiff's polybase according to claim 20, characterised in that P′ is the n-valent radical of a polypeptide P selected from among the glycosylated or unglycosylated peptide hormones, the glycosylated or unglycosylated toxins, the enzymes and the enzyme inhibitors.

**22.** Schiff's polybase according to claim 20 or 21, characterised by formula IV above, where Oside represents a radical of a glycosaminoglycane Oside-Z selected from among heparin, heparin fragments, chondroitin sulphate 4-S, chondroitin sulphate 6-S, heparan sulphate and dermatane sulphate, deprived of the terminal reducing unit Z.

**23.** Schiff's polybase according to claim 20, characterised by formula IV wherein
- Z′ is the terminal reducing saccharide unit of a glycosaminoglycane of the heparin Oside-Z type in its aldehyde form obtained from the nitrous depolymerisation of heparin, said Oside-Z consisting of a mixture of heparin fragments having a mean molecular mass between 2000 and 8000 daltons;
- Oside is the residue of the polysaccharide Oside-Z.

**24.** Schiff's polybase according to claim 20, characterised by formula IV wherein P′ and n are as defined in claim 20;
- Z′ is the terminal reducing saccharide unit of a glycosaminoglycane of the heparin Oside-Z type in its aldehyde form obtained from the nitrous depolymerisation of heparin, said Oside-Z consisting of a mixture of heparin fragments having a mean molecular mass of about 4500 daltons;
- Oside is the residue of the polysaccharide Oside-Z.

**25.** Schiff's polybase according to any of claims 20 to 24, characterised by formula IV wherein Z′ has the structure

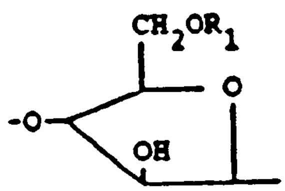

wherein $R_1$ is hydrogen or an $SO_3^-$ group.

**26.** Schiff's polybase according to any one of claims 20 to 25, characterised by formula (IV) wherein
- P′ is the n-valent radical of a thrombolytic enzyme linked to the n NH groups obtained from n free amino groups originally present in the molecule of said thrombolytic enzyme;
- n is from 2 to 10.

**27.** Schiff's polybase according to claim 26,
characterised by formula (IV) wherein
- P′ is the n-valent radical of urokinase linked to the n NH groups obtained from n free amino groups originally present in the molecule of said urokinase;
- n is less than or equal to 6, preferably 6, 4 or 3.

**28.** Schiff's polybase according to claim 26,
characterised by formula (IV) wherein
- P′ is the n-valent radical of streptokinase linked to the n NH groups obtained from n free amino groups originally present in the molecule of said streptokinase,
- n is from 2 to 4.

**29.** Schiff's polybase according to claim 26, characterised by formula (IV) wherein

- P′ is the n-valent radical of tPA linked to the n NH groups obtained from n free amino groups originally present in the molecule of said tPA;
- n is from 3 to 10.

**30.** Schiff's polybase of formula

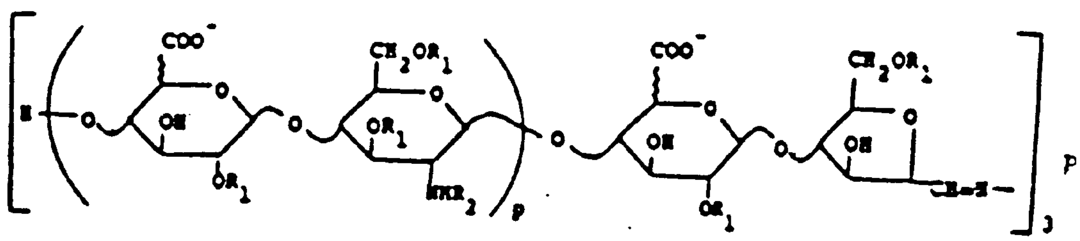

wherein

- $R_1$ is hydrogen or an $SO_3^-$ group,
- $R_2$ is an acetyl group or an $SO_3^-$ group,
- P′ is the trivalent radical of urokinase linked to the 3 NH groups obtained from 3 free amino groups originally present in the molecule of urokinase and
- p, denoting from 4 to 24, represents the number of disaccharide units of heparin fragments obtained by nitrous depolymerisation in a mixture having a mean molecular mass of about 4500 daltons and containing the site for fixing to AT III.

**31.** Schiff's polybase of formula

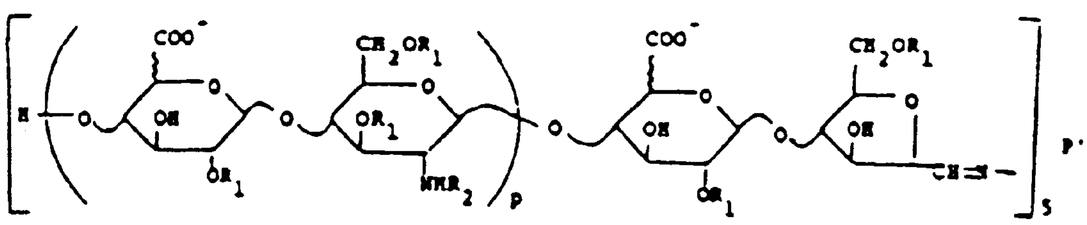

wherein

- $R_1$ is hydrogen or an $SO_3^-$ group,
- $R_2$ is an acetyl group or an $SO_3^-$ group,
- P′ is the pentavalent radical of tPA linked to the 5 NH groups obtained from 5 free amino groups originally present in the molecule of tPA, and
- p, from 4 to 24, represents the number of disaccharide units of heparin fragments obtained by nitrous depolymerisation in a mixture having a mean molecular mass of about 4500 daltons and containing the site for fixing to AT III.

**32.** Schiff's polybase of formula

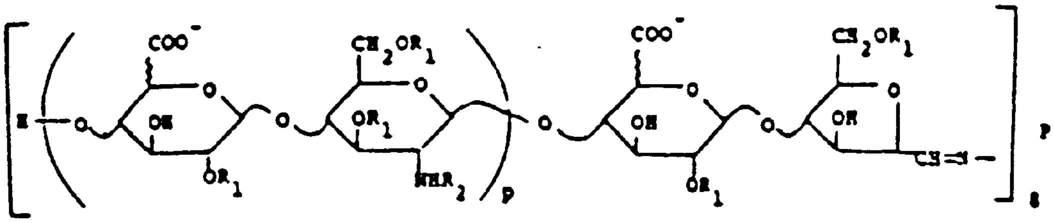

dans laquelle

wherein

- $R_1$ is hydrogen or an $SO_3^-$ group,
- $R_2$ is an acetyl group or an $SO_3^-$ group,
- P′ is the octovalent radical of tPA linked to the 8 NH groups obtained from 8 free amino groups originally present in the molecule of tPA, and
- p, denoting from 4 to 24, represents the number of disaccharide units of heparin fragments obtained by nitrous depolymerisation in a mixture having a mean molecular mass of about 4500 daltons and containing the site for fixing to AT III.

**33.** Schiff's polybase of formula

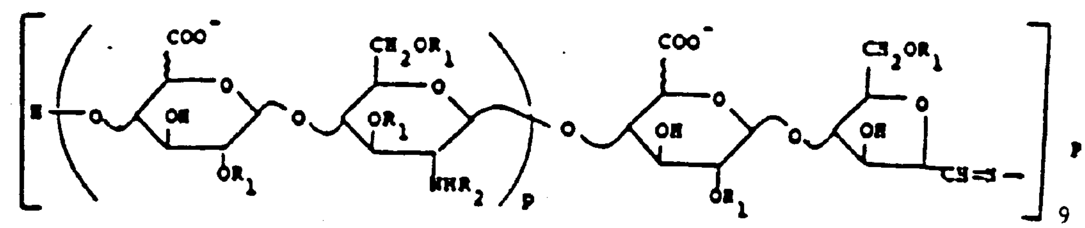

wherein

- $R_1$ is hydrogen or an $SO_3^-$ group,
- $R_2$ is an acetyl group or an $SO_3^-$ group,
- P′ is the monovalent radical of tPA linked to 9 NH groups obtained from 9 free amino groups originally present in the molecule of the tPA, and
- p, denoting from 4 to 24, represents the number of disaccharide units of heparin fragments obtained by nitrous depolymerisation in a mixture having a mean molecular mass of about 4500 daltons and containing the site for fixing to AT III.

**34.** Pharmaceutical composition, characterised in that it contains as active substance an effective amount of at least one N-polyosyl-polypeptide according to any of claims 1 to 14 in conjunction with a pharmaceutical carrier.

**35.** Biological reagent comprising a product according to any one of claims 1 to 14.

**Claims for the following Contracting State : ES**

**1.** Process for preparing N-polyosyl-polypeptide of formula

$$(\text{Oside-Z'-CH}_2\text{-NH-})_n\text{P'} \quad (I)$$

wherein

- P′ is the n-valent radical of a polypeptide P linked to n NH groups coming from n free amino groups originally present in the P molecule, P′ being other than the monovalent radical of haemoglobin;
- Z′ is the terminal reducing saccharide unit of the polysaccharide Oside-Z in its aldehyde form, either natural or produced by nitrous depolymerisation, deprived of the aldehyde group of Z;
- Oside is the residue of the polysaccharide Oside-Z;
- n is an integer from 2 to 30;

or, optionally, one of the pharmaceutically acceptable salts thereof, characterised in that a polypeptide P represented by the formula

$$P°(NH_2)_m \qquad (II)$$

wherein P° is the m-valent radical of the polypeptide P to which its m functional free $NH_2$ groups are linked, said m functional $NH_2$ groups being the terminal amino group and the amino groups in the epsilon position of the (m-1) lysines contained in the polypeptide, is treated with an excess of a polyoside represented by the formula

$$\text{Oside-Z} \qquad (III)$$

wherein Z is the terminal reducing saccharide unit of said polysaccharide in its aldehyde form which may either be natural or produced by nitrous depolymerisation and Oside is the residue of the chain of said polysaccharide, and the Schiff's polybase obtained of formula

$$(\text{Oside-Z'-CH=N-})_n\, P' \qquad (IV)$$

wherein the monovalent radical Oside-Z'- represents the polysaccharide Oside-Z as defined above, deprived of the aldehyde group of Z, and P' and n are as defined in claim 1, is subjected to reduction with a cyanoborohydride.

2. Process according to claim 1, characterised in that the starting polyoside Oside-Z used consists of a mixture of heparin fragments obtained by nitrous depolymerisation having a mean molecular mass of between 2000 and 8000 daltons.

3. Process according to any one of claims 1 and 2, characterised in that the starting polyoside Oside-Z used consists of a mixture of heparin fragments obtained by nitrous depolymerisation having a mean molecular mass of about 4500 daltons.

4. Process according to any one of claims 1 to 3, characterised in that the cyanoborohydride used is sodium cyanoborohydride.

5. Process according to any one of claims 1 to 4, characterised in that the cyanoborohydride used is sodium cyanoborohydride, the reaction between $P°(NH_2)_m$ and Oside-Z being performed in the presence of said sodium cyanoborohydride so that the final N-polyosylpolypeptide is obtained directly.

6. Process according to claim 1, characterised in that in formula (I) P' is the n-valent radical of a polypeptide P selected from among glycosylated or unglycosylated peptide hormones, glycosylated or unglycosylated toxins, enzymes and enzyme inhibitors.

7. Process according to claim 1, characterised in that in formula (I) Oside represents the radical of a glycosaminoglycane Oside-Z selected from heparin, heparin fragments, chondroitin sulphate 4-S, chondroitin sulphate 6-S, heparan sulphate and dermatan sulphate, deprived of the terminal reducing unit Z.

8. Process according to any of claims 1 to 7, characterised in that formula (I) Z' has the structure

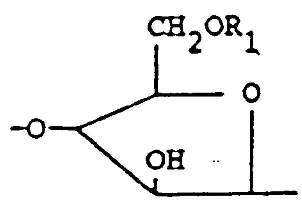

wherein $R_1$ is hydrogen or an $SO_3^-$ group.

9. Process according to any one of claims 1 to 8, characterised in that, in formula (I)
- P' is the n-valent radical of a thrombolytic enzyme linked to n NH groups obtained from n free amino groups originally present in the molecule of said thrombolytic enzyme;
- n is from 2 to 10.

10. Process according to claim 1, characterised in that in formula (I)
- P' is the n-valent radical of urokinase linked to n NH groups obtained from n free amino groups originally present in the molecule of said urokinase;

- n is less than or equal to 6, preferably 6, 4 or 3.

**11.** Process according to claim 1, characterised in that in formula (I)
- P′ is the n-valent radical of streptokinase linked to the n NH groups obtained from n free amino groups originally present in the molecule of said streptokinase,
- n is from 2 to 4.

**12.** Process according to claim 1, characterised in that in formula (I)
- P′ is the n-valent radical of tPA linked to the n NH groups obtained from n free amino groups originally present in the molecule of said tPA;
- n is from 3 to 10.

**13.** Process according to claim 1, characterised in that an N-polyosyl-polypeptide of formula

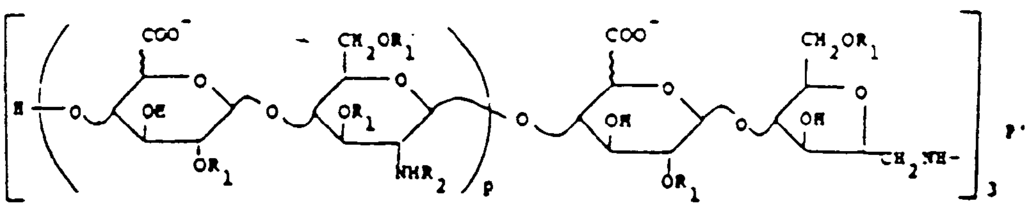

is prepared, wherein
- $R_1$ is hydrogen or an $SO_3^-$ group,
- $R_2$ is an acetyl group or an $SO_3^-$ group,
- P′ is the trivalent radical of urokinase linked to the 3 NH groups obtained from 3 free amino groups originally present in the molecule of urokinase, and
- p, denoting from 4 to 24, represents the number of disaccharide units of heparin fragments obtained by nitrous depolymerisation in a mixture having a mean molecular mass of about 4500 daltons and containing the site for fixing to AT III.

**14.** Process according to claim 1, characterised in that an N-polyosyl-polypeptide of formula

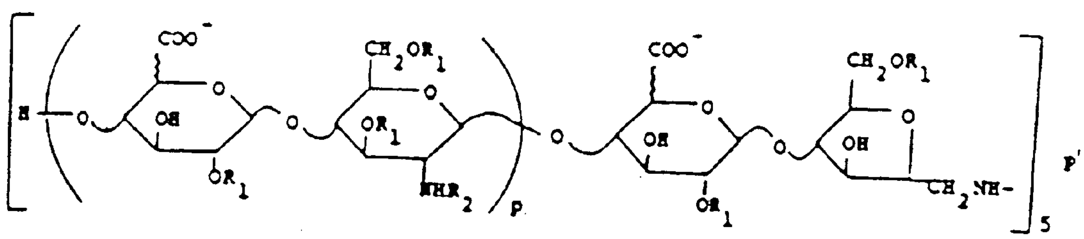

is prepared wherein
- $R_1$ is hydrogen or an $SO_3^-$ group,
- $R_2$ is an acetyl group or an $SO_3^-$ group,
- P′ is the pentavalent radical of tPA linked to the 5 NH groups obtained from 5 free amino groups originally present in the molecule of tPA, and
- p, denoting from 4 to 24, represents the number of disaccharide units of heparin fragments obtained from nitrous depolymerisation in a mixture having a molecular mass of about 4500 daltons and containing the site for fixing to AT III.

**15.** Process according to claim 1, characterised in that an N-polyosyl-polypeptide of formula

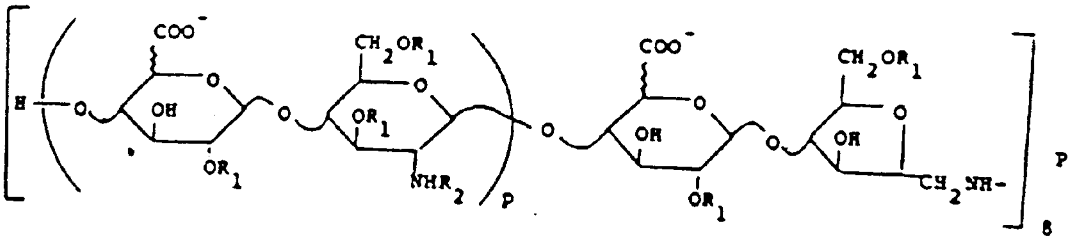

is prepared wherein

- $R_1$ is hydrogen or an $SO_3^-$ group,
- $R_2$ is an acetyl group or an $SO_3^-$ group,
- P′ is the octovalent radical of tPA linked to the 8 NH groups obtained from 8 free amino groups originally present in the molecule of tPA, and
- p, denoting from 4 to 24, represents the number of disaccharide units of heparin fragments obtained by nitrous depolymerisation in a mixture having a molecular mass of about 4500 daltons and containing the site for fixing to AT III.

**16.** Process according to claim 1, characterised in that an N-polyosyl-polypeptide of formula

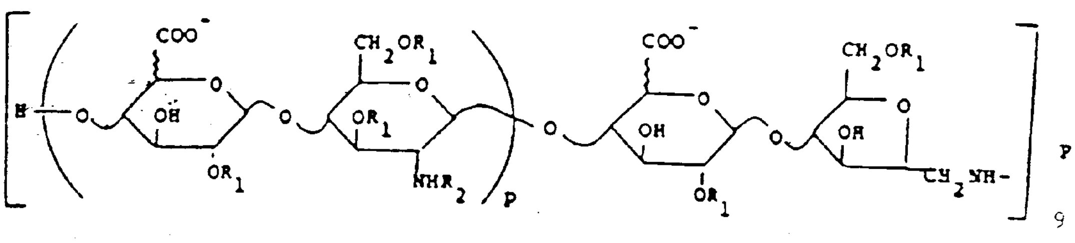

is prepared wherein

- $R_1$ is-hydrogen or an $SO_3^-$ group,
- $R_2$ is an acetyl group or an $SO_3^-$ group,
- P′ is the nonavalent radical of tPA linked to the 9 NH groups obtained from 9 free amino groups originally present in the molecule of tPA, and
- p, denoting from 4 to 24, represents the number of disaccharide units of heparin fragments obtained by nitrous depolymerisation in a mixture having a molecular mass of about 4500 daltons and containing the site for fixing to AT III.

**17.** Biological reagent comprising a product obtained by the process according to any one of claims 1 to 16.